(19)

Europäisches
Patentamt
European
Patent Office
Office européen
des brevets

(11) **EP 3 061 494 A1**

(12) **EUROPEAN PATENT APPLICATION**

(43) Date of publication:
**31.08.2016 Bulletin 2016/35**

(51) Int Cl.:
***A61N 2/02*** (2006.01)

(21) Application number: **16156921.5**

(22) Date of filing: **23.02.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.02.2015 JP 2015039334**
**27.02.2015 JP 2015039337**
**27.02.2015 JP 2015039340**
**27.02.2015 JP 2015039344**

(71) Applicant: **Nihon Kohden Corporation**
**Shinjuku-ku**
**Tokyo**
**161-8560 (JP)**

(72) Inventors:
• **SAKAKI, Takuya**
**Tokyo, 161-8560 (JP)**

• **HONMA, Motohisa**
**Tokyo, 161-8560 (JP)**
• **OTAKA, Kohei**
**Tokyo, 161-8560 (JP)**
• **SATO, Takanori**
**Tokyo, 161-8560 (JP)**
• **KANESHIRO, Akira**
**Tokyo, 161-8560 (JP)**
• **HARADA, Hiroshi**
**Tokyo, 161-8560 (JP)**
• **KUMADA, Yasuhiro**
**Tokyo, 161-8560 (JP)**
• **YOKOYAMA, Hiroshi**
**Tokyo, 161-8560 (JP)**

(74) Representative: **McCartney, Jonathan William**
**Haseltine Lake LLP**
**Redcliff Quay**
**120 Redcliff Street**
**Bristol BS1 6HU (GB)**

(54) **LINK MECHANISM FOR ARM PORTION**

(57)     A link mechanism for an armportion includes an armportion, a non-rotating arm attachment portion, a variable-length rod, and a fixed-length rod. The arm portion holds a medical instrument. The arm portion is attached to the arm attachment portion to be rotatable about an arm rotational axis as a center. The variable-length rod includes an elastic member which biases an elastic force to bring about a recovery against a pressing force. One end of the variable-length rod is pivotably attached to the arm attachment portion and the other end of the variable-length rod is pivotably connected to one end of the fixed-length rod. The fixed-length rod has its other end attached to the arm portion to be movable about the arm rotational axis upon rotation of the arm portion. When the center of gravity of the arm portion is horizontally aligned with the arm rotational axis, the direction of action of the elastic force is set so as to be matched with a tangential direction to the rotational trajectory along which the other end of the fixed-length rod is movable.

EP 3 061 494 A1

**Description**

**BACKGROUND**

The present invention relates to a link mechanism for an arm portion.

Conventionally, when a medical instrument is supported at one end of a rotatable arm mounted in a medical apparatus, a counterweight is attached to the other end of the arm to balance the weight of the entire arm, as disclosed in Japanese Patent Publication No. 2011-224387. Balancing the weight of the entire arm makes it possible to hold the medical instrument at a predetermined position and operate the arm free from a sense of the weight of the medical instrument.

**SUMMARY**

However, in an arrangement having a counterweight attached to the other end of the arm, the medical apparatus is relatively large and is therefore comparatively heavy. Although a spring is often used in place of a counterweight, it is difficult in this scheme to ensure a given balance because the holding torque varies depending on the arm angle.

The present invention has been made in order to solve such problems. In other words, it is an object to provide a link mechanism for an arm portion that can contribute achieving a small, lightweight medical apparatus and facilitating arm balancing.

A link mechanism for an armportion includes an armportion, a non-rotating arm attachment portion, a variable-length rod, and a fixed-length rod. The arm portion holds a medical instrument. The arm portion is attached to the arm attachment portion to be rotatable about an arm rotational axis as a center. The variable-length rod has a variable total length and includes an elastic member which biases an elastic force to turn back against a pressing force. The fixed-length rod has a fixed total length. One end of the variable-length rod is pivotally attached to the arm attachment portion and the other end of the variable-length rod is pivotally connected to one end of the fixed-length rod. The fixed-length rod has its other end attached to the arm portion to be movable about the arm rotational axis upon rotation of the arm portion. When the center of gravity of the arm portion is horizontally aligned with the arm rotational axis, the direction of action of the elastic force is set so as to be matched with a tangential direction to the rotational trajectory along which the other end of the fixed-length rod is movable.

The medical instrument preferably includes a magnetic stimulation coil used in a magnetic stimulation treatment which magnetically stimulates a living body.

**BRIEF DESCRIPTION OF THE DRAWINGS**

Fig. 1 is a side view illustrating a magnetic stimulation treatment system.
Fig. 2 is a side view illustrating the main part of a magnetic stimulation apparatus illustrated in Fig. 1.
Fig. 3A (A) illustrates a plan view of a posture locking device for a medical instrument while the posture of the medical instrument is kept locked.

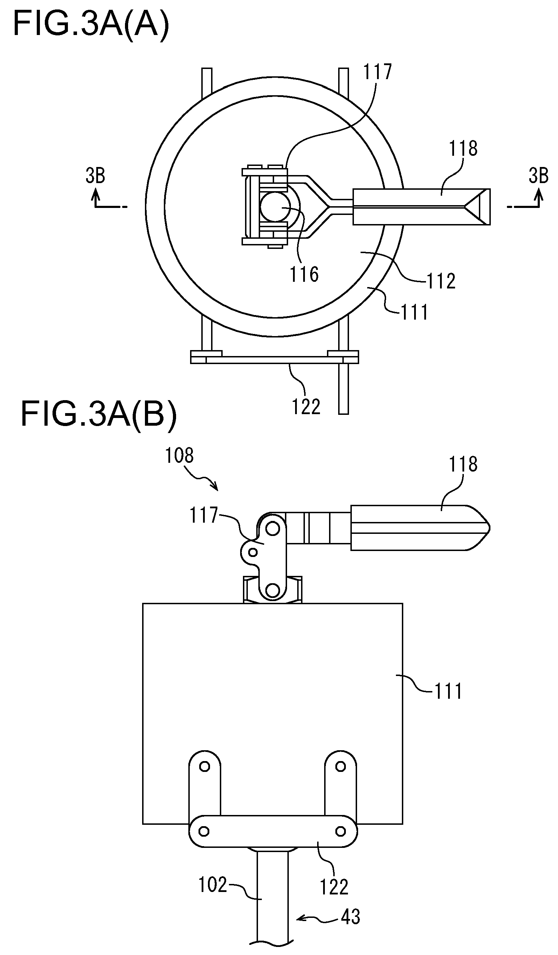

Fig. 3A (B) illustrates a front view of a posture locking device for a medical instrument while the posture of the medical instrument is kept locked.

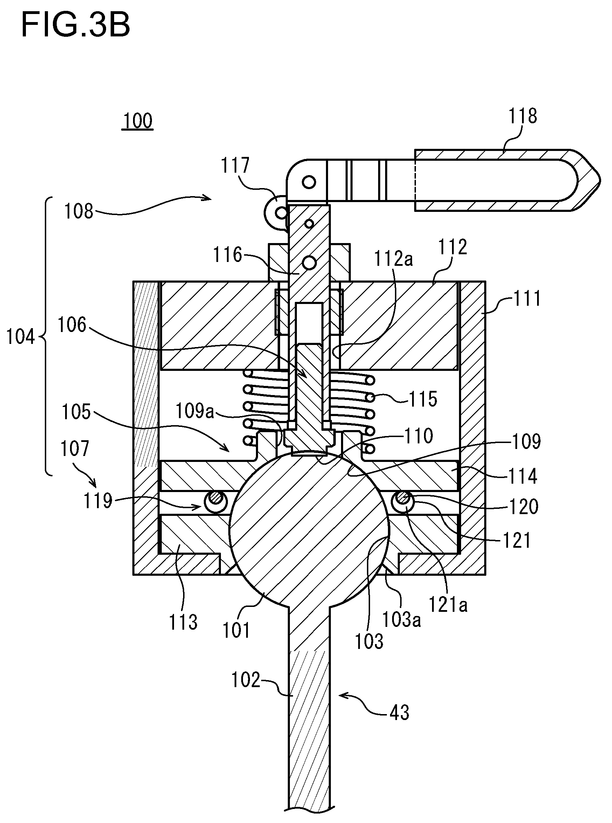

Fig. 3B is a sectional view taken along a line 3B - 3B in Fig. 3A(A).

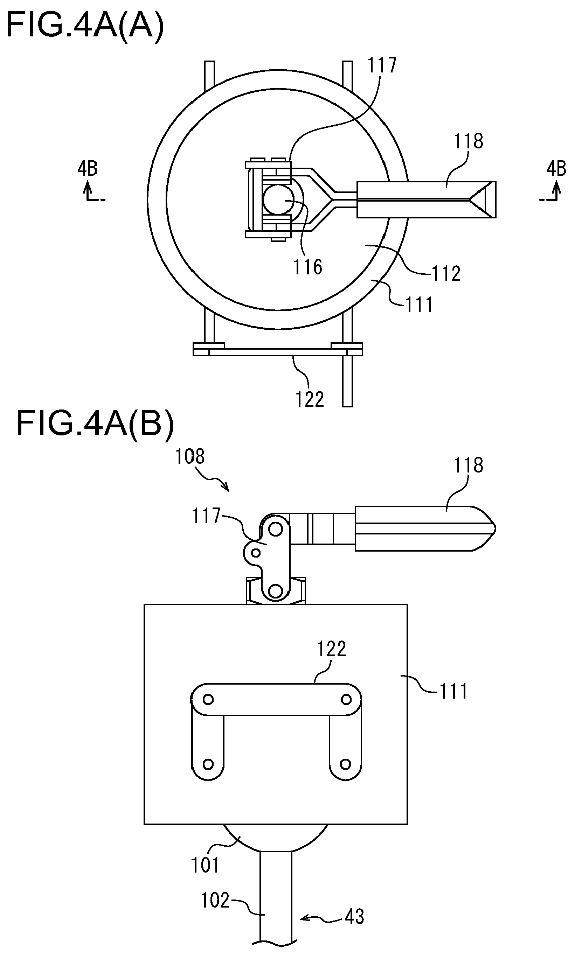

Fig. 4A (A) illustrates a plan view of the posture locking device for a medical instrument while the posture of the medical instrument is adjusted finely.
Fig. 4A (B) illustrates a front view of the posture locking device for a medical instrument while the posture of the medical instrument is adjusted finely.

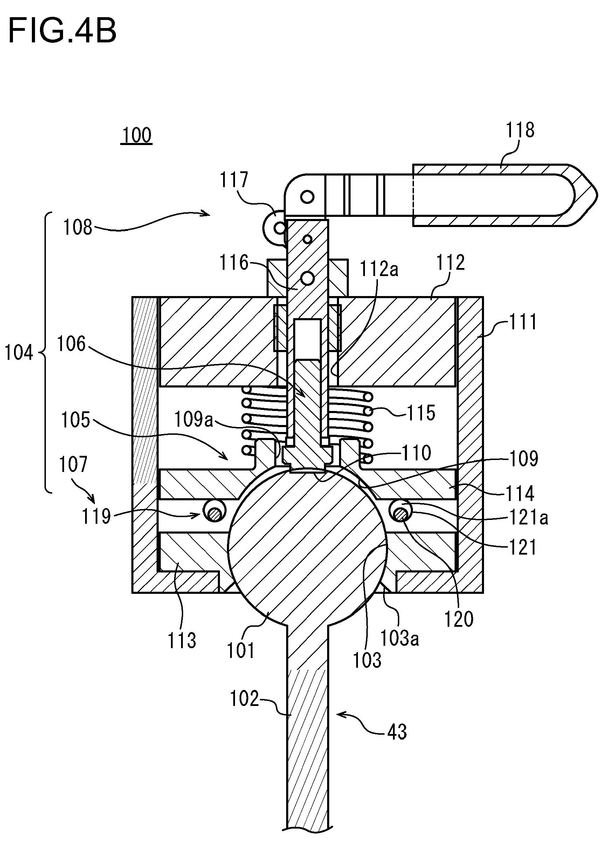

Fig. 4B is a sectional view taken along a line 4B - 4B in Fig. 4A(A).

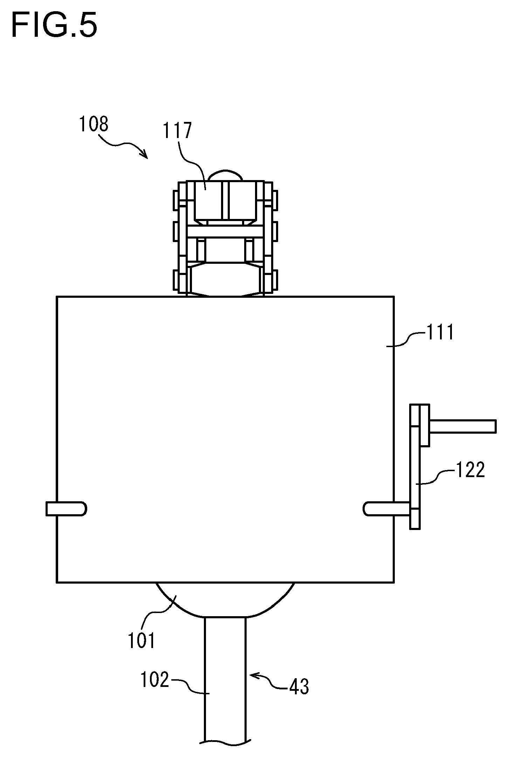

Fig. 5 is a side view illustrating the posture locking device for a medical instrument while the posture of the medical instrument is adjusted finely.

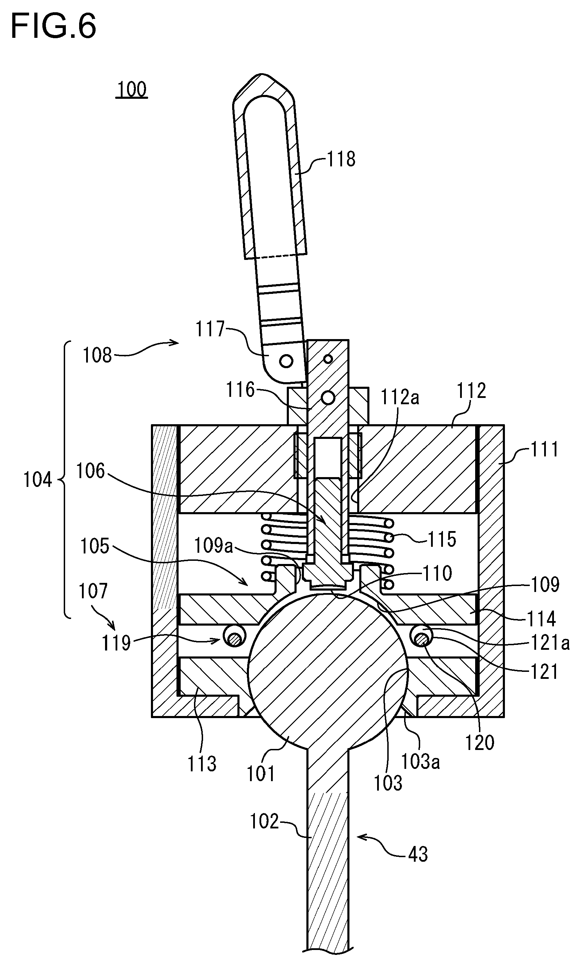

Fig. 6 is a sectional view illustrating the posture locking device for a medical instrument when biasing with a first resistive force by a first braking portion and biasing with a second resistive force by a second braking portion have been cancelled.

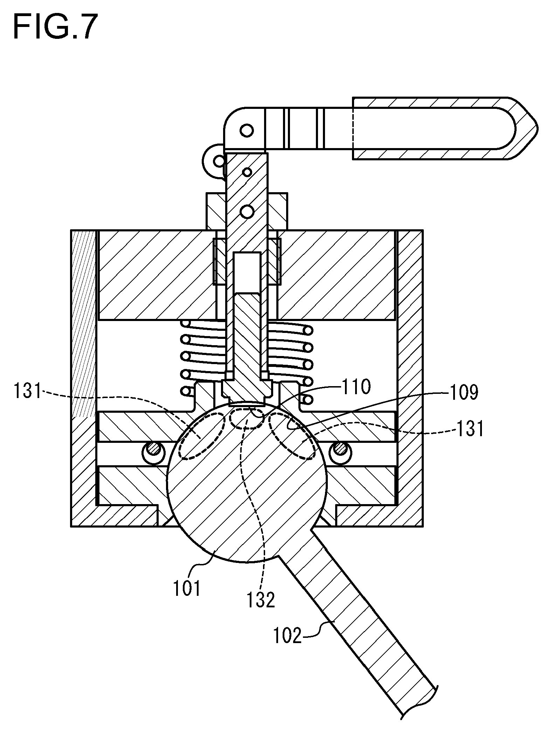

Fig. 7 is a sectional view illustrating a modification in which the braking force to lock the posture of the medical instrument is increased.

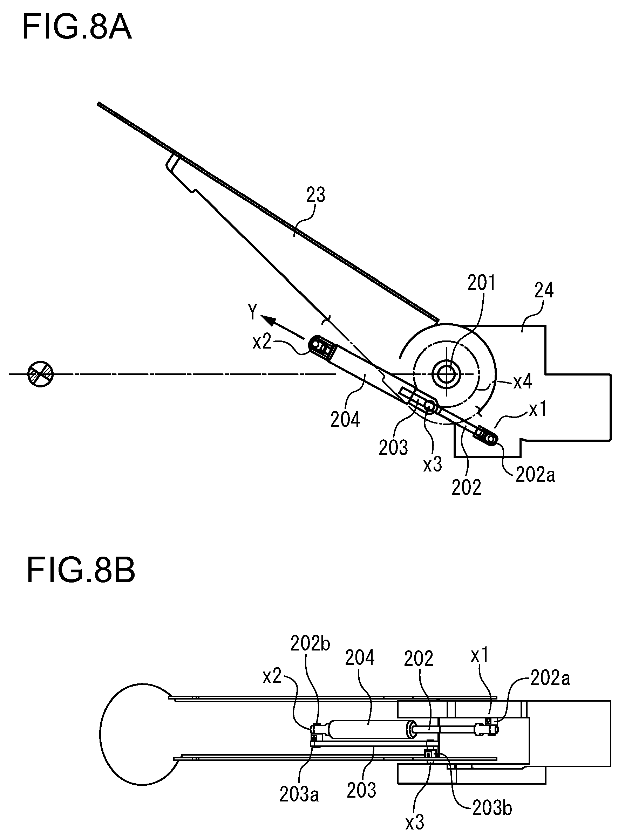

Fig. 8A illustrates a side view of the link mechanism for an arm portion according to the embodiment.
Fig. 8B illustrates a bottom view of the link mechanism for an arm portion according to the embodiment.

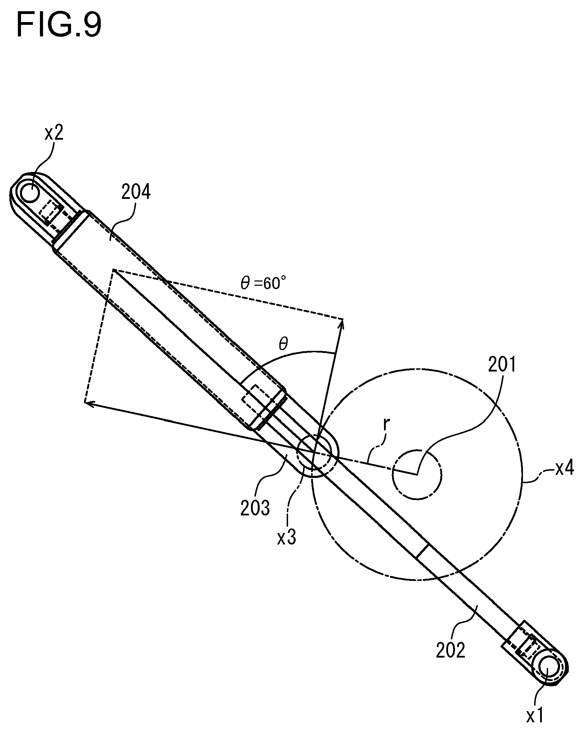

Fig. 9 is a view for explaining an effect produced in the direction of action of an elastic force.

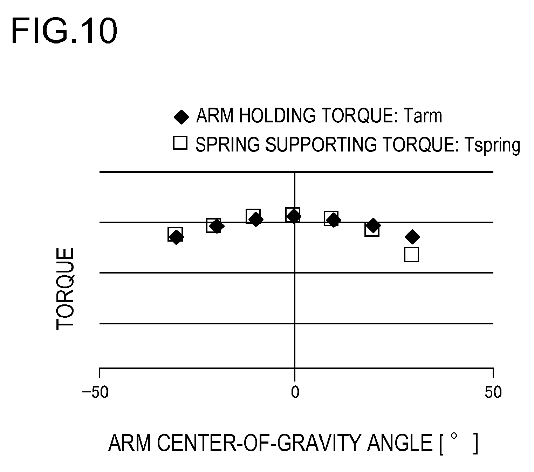

Fig. 10 is a graph illustrating an example of an arm torque diagram in the embodiment.

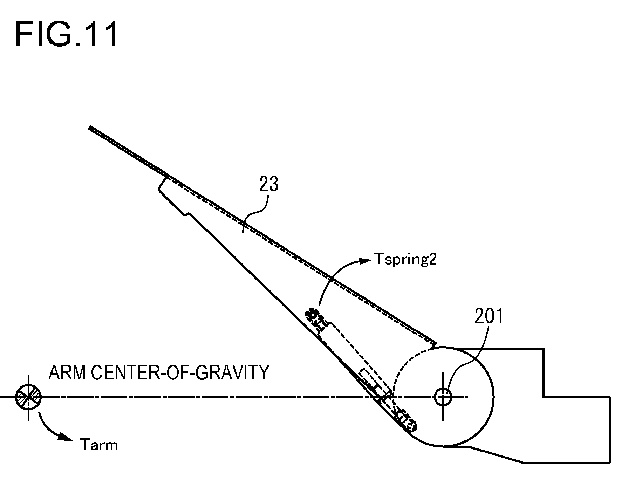

Fig. 11 is a side view illustrating a link mechanism for an arm portion according to a comparative example in which only a spring is used.

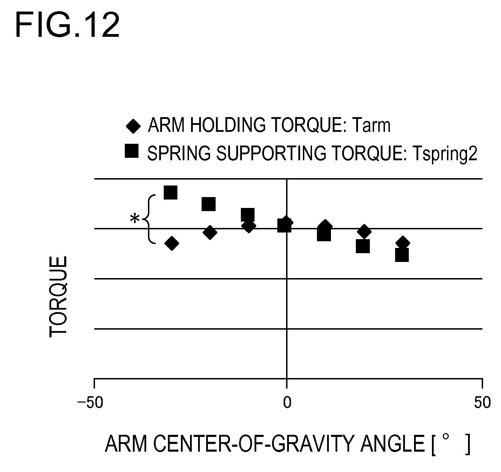

Fig. 12 is a graph illustrating an example of an arm torque diagram in the comparative example.

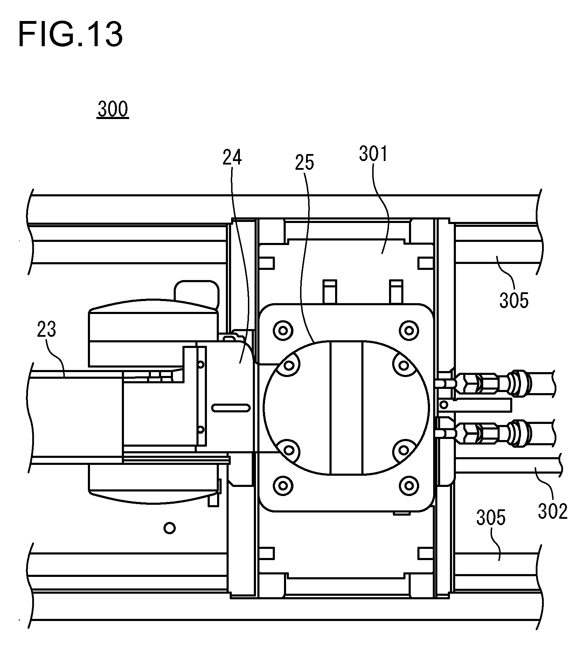

Fig. 13 is a plan view illustrating a sliding device for a medical apparatus.

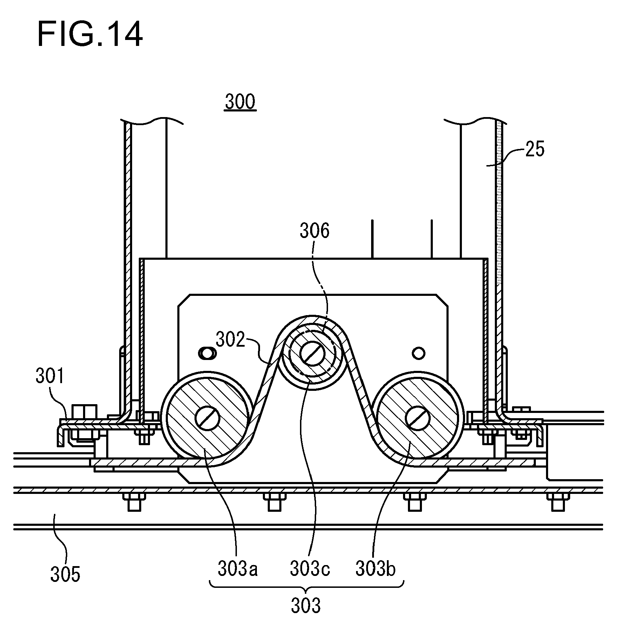

Fig. 14 is a sectional view illustrating the main part of the internal structure of a post portion in the sliding device for a medical apparatus.

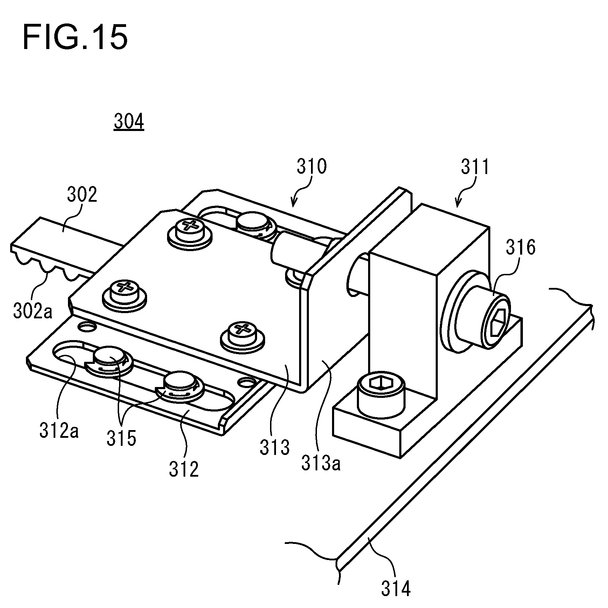

Fig. 15 is a perspective view illustrating a tension applying member.

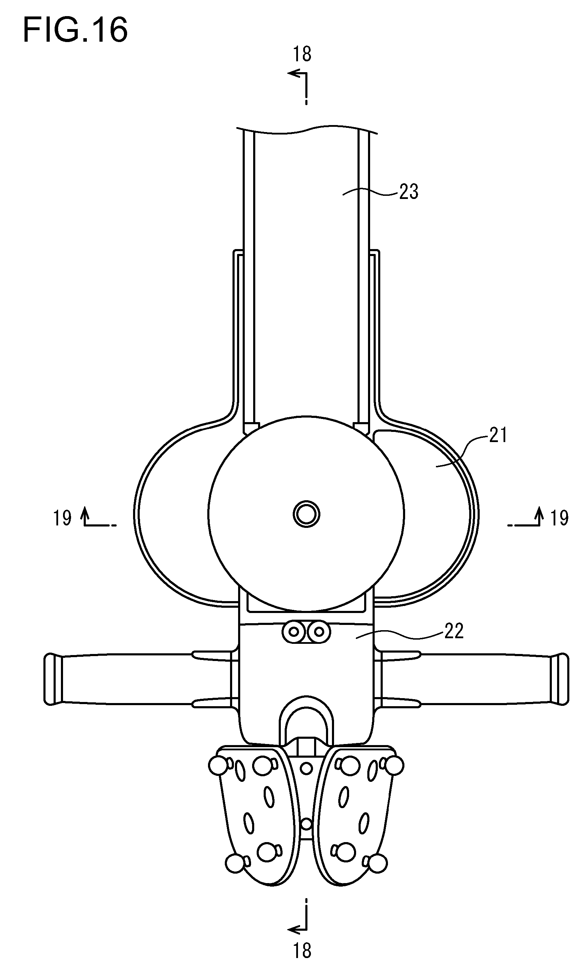

Fig. 16 is a view illustrating the distal end portion of the arm portion in the magnetic stimulation apparatus when viewed from above.

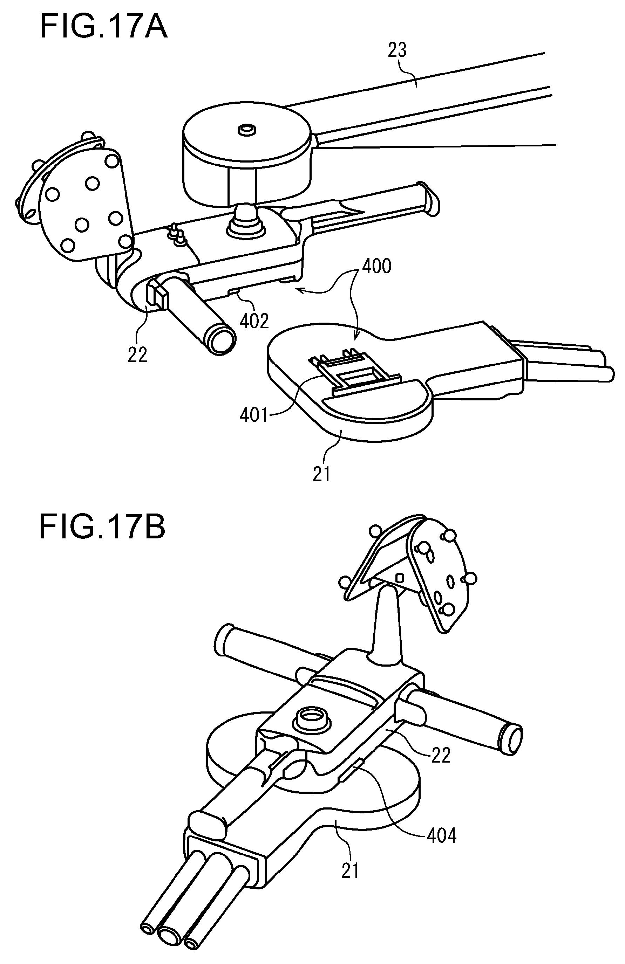

Fig. 17A illustrates a perspective view of the armportion, a coil attachment portion, and a magnetic stimulation coil in the magnetic stimulation apparatus before the magnetic stimulation coil is attached to the coil attachment portion

Fig. 17B illustrates a perspective view of the armportion, the coil attachment portion, and the magnetic stimulation coil in the magnetic stimulation apparatus after the magnetic stimulation coil is attached to the coil attachment portion, when viewed in a direction different from that shown in Fig. 17A.

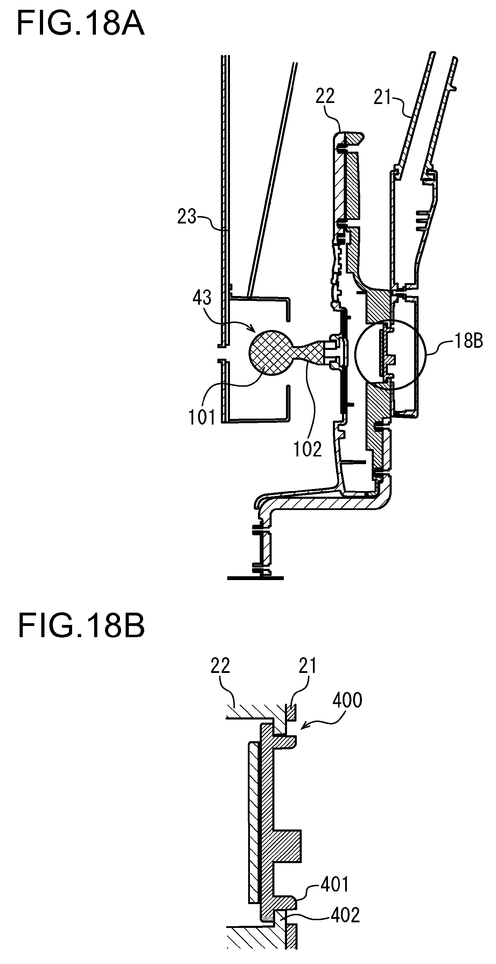

Fig. 18A illustrates a sectional view taken along a line 18 - 18 in Fig. 16andFig. 18B illustrates an enlarged sectional view of a portion denoted by reference numeral 18B in Fig. 18A.

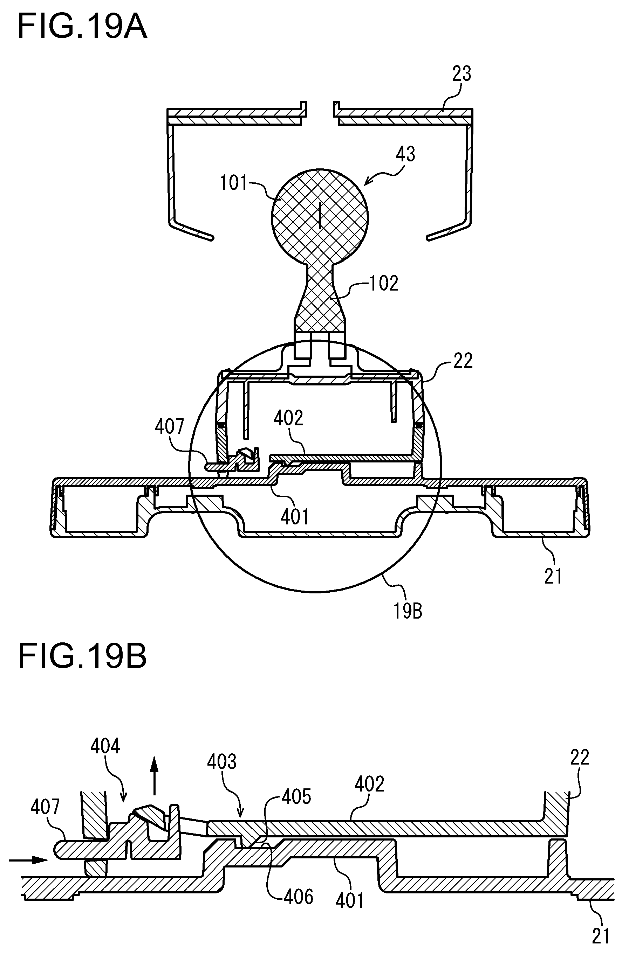

Fig. 19A illustrates a sectional view taken along a line 19 - 19 in Fig. 16.

Fig. 19B illustrates an enlarged sectional view of a portion denoted by reference numeral 19B in Fig. 19A.

## DETAILED DESCRIPTION

(Embodiment)

An embodiment of the present invention will be described below with reference to the accompanying drawings. The following description is intended to limit neither the technical scope nor the meanings of terms defined in "What is claimed is." The dimensional ratios of the drawings may be exaggerated for the sake of descriptive convenience and different from actual ones.

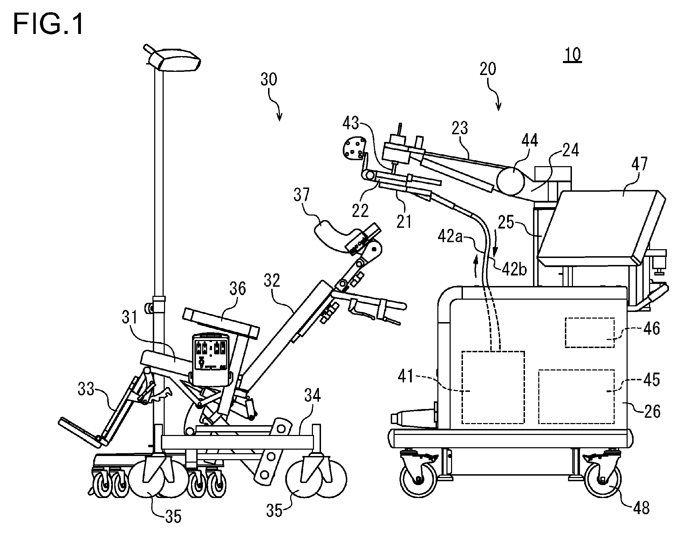

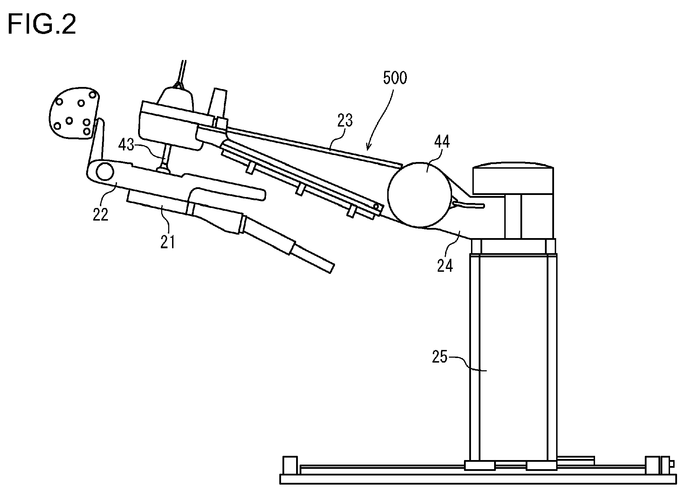

Fig. 1 is a side view illustrating a magnetic stimulation treatment system 10, and Fig. 2 is a side view illustrating the main part of a magnetic stimulation apparatus 20 illustrated in Fig. 1.

The magnetic stimulation treatment system 10 illustrated in Fig. 1 is used in a therapeutic method for directly stimulating a brain, as one type of therapy for rehabilitation or the like after cerebral apoplexy or neurological disease such as Parkinson's disease and depression. As a therapeutic method for directly stimulating a brain, a magnetic stimulation treatment which magnetically stimulates a specific site in the head of a patient, such as rTMS (Repetitive transcranial magnetic stimulation), is employed. The magnetic stimulation treatment is used to induce an electric field into the skull to stimulate nerves and has a variety of features, including attaining tissue stimulation without dissipating magnetic energy and stimulating a brain more accurately and reliably.

Referring to Fig. 1, the magnetic stimulation treatment system 10 includes a magnetic stimulation apparatus 20 and a seat 30. The magnetic stimulation apparatus 20 stimulates the brain of a patient with magnetic energy. The patient sits on the seat 30 while undergoing therapy.

The seat 30 includes a seating surface portion 31, a backrest portion 32, a foot support portion 33, a base portion 34 which supports the seating surface portion 31, and casters 35 mounted on the lower surface of the base portion 34. The angles of inclination of the seating surface portion 31, the backrest portion 32, and the foot support portion 33 can be adjusted in accordance with the posture of the patient. Armrests 36 are attached to the seating surface portion 31 on its lateral sides. A headrest 37 which fixes the head of the patient is attached to the top of the backrest portion 32. The casters 35 can move the seat 30.

The magnetic stimulation apparatus 20 includes a magnetic stimulation coil 21 (to be also simply referred to as the "coil 21" hereinafter) as a medical instrument, a coil attachment portion 22, an arm portion 23, an arm attachment portion 24, a post portion 25, and a cart portion 26. The coil attachment portion 22 holds the coil 21 attached on its lower surface. The armportion 23 supports the coil attachment portion 22 suspended from it. The arm attachment portion 24 holds the proximal end portion of the arm portion 23. The post portion 25 supports the arm attachment portion 24. The cart portion 26 holds the post portion 25. The medical instrument is not limited to the coil 21 and widely includes instruments, requiring posture adjustment, which are used in a therapeutic device, a diagnostic device, a research device, an inspection device, and the like.

The coil 21 magnetically stimulates the head of a living body (patient). Coil elements built into the coil 21 can have a circular, figure-of-eight, or conical shape as appropriate. Since the shape of a magnetic field applied to the head varies in each individual shape of the coil elements, a suitable shape is selected in accordance with, for example, the type of neurological disorder. Because a high current on the order of several thousand amperes is supplied to the coil elements, a cooling unit (not illustrated) which cools the coil elements surrounds the coil elements. The coil 21 is detachably attached to the coil attachment portion 22. The coil 21 is replaced with a new coil 21 including coil elements with a different shape, in accordance with kinds of a treatment. The cooling unit for the coil 21 is connected to a chiller 41 built into the cart portion 26. The chiller 41 supplies a cold refrigerant to the cooling unit through a supply hose 42a. The refrigerant having cooled the coil elements is returned from the cooling unit to the chiller 41 through a discharge hose 42b.

The arm portion 23 supports the coil attachment portion 22 suspended from the lower surface of its distal end through a ball joint 43. The posture of the coil attachment portion 22 relative to the arm portion 23 can be adjusted to a desired posture by the ball joint 43. The proximal end portion of the arm portion 23 is attached to the arm attachment portion 24 through an articulatedportion 44. The articulatedportion 44 can pivot the arm portion 23 in a vertical plane. The arm attachment portion 24 is attached to the post portion 25 through a bearing portion (not illustrated). The bearing portion can pivot the arm attachment portion 24 in a horizontal plane. The post portion 25 is set on the upper surface of the cart portion 26 to be slidable in the direction coming close to or moving away from the patient (the horizontal direction on the sheet surface of Fig. 1) through a sliding mechanism. The arm portion 23, the arm attachment portion 24, and the post portion 25 constitute an arm assembly 500. The arm assembly 500 slides through the aforementioned sliding mechanism.

The posture of the coil 21 relative to the head of the patient can be adjusted to a desired posture by adjusting the posture of the coil attachment portion 22 relative to the arm portion 23, pivoting the arm portion 23 in a vertical plane, pivoting the arm attachment portion 24 in a horizontal plane, and sliding the post portion 25. A magnetic stimulus needs to be precisely applied to a specific site in the head of the patient. Therefore, once the posture of the coil 21 has been adjusted, the ball joint 43, the articulated portion 44, the bearing portion, and the sliding mechanism are locked in an inoperable state. This accurately positions the coil 21.

The cart portion 26 houses not only the chiller 41 but also a current supply device 45 and a controller 46. The current supply device 45 accommodates electrical components for supplying a high current to the coil elements. The controller 46 controls the operation of the entire magnetic stimulation treatment system 10. The temperatures of the coil elements, signals from the chiller 41, signals from the current supply device 45, vital sign signals obtained from a living body, and the like are input to the controller 46 via an input/output unit. Examples of the vital sign signals may include electrocardiographic signals of the patient (to generate electrocardiographic data), the transmittances of the blood flowing through the fingertips of the patient for red light and infrared light (to measure an arterial oxygen saturation (to be referred to as SpO2 hereinafter)), and myoelectric signals of the living body (to generate electromyographic data). Signals for controlling the operation of the chiller 41, signals for controlling the operation of the current supply device 45, and the like are output from the controller 46 via the input/output unit. The cart portion 26 holds a monitor 47 connected to the controller 46. The monitor 47 displays the operating state of the current supply device 45, the state of the patient, and the like. The controller 46 uses the vital sign signals to control activation of the current supply device 45 and, in turn, to control the intensity of a magnetic stimulus applied to the living body by the coil 21.

The cart portion 26 has casters 48 mounted on its lower surface. The casters 48 can move the cart portion 26 and, by extension, the magnetic stimulation apparatus 20.

To be brief, a magnetic stimulation treatment is performed using the magnetic stimulation treatment system 10 as follows.

First, before the start of therapy by a magnetic stimulation treatment, the operator precisely sets the position of the coil 21 to a position suitable for treating symptoms in the patient. It can be checked by display (in a site subjected to a magnetic stimulus) on the monitor 47 whether the position of the coil 21 is appropriate.

When therapy is started, the controller 46 controls the current supply device 45 to supply a current to the coil 21. An electric field is induced from the coil 21 into the skull of the patient to magnetically stimulate his or her head. Such magnetic stimulation is performed to treat neurological disease such as Parkinson's disease. The controller 46 controls the intensity of a magnetic stimulus by changing the magnitude of a current output to the coil 21 and the interval and time to supply a current.

The controller 46 displays electrocardiographic data, SpO2, electromyographic data, and the like on the monitor 47, based on vital sign signals (electrocardiographic signals, transmittances, and myoelectric signals) of the patient. The operator can easily determine the therapeutic situation and the condition of the patient during therapy by observing these types of displayed information. The operator suitably adjusts the intensity of a magnetic stimulus in accordance with the state of the patient while monitoring this state.

The magnetic stimulation treatment requires precisely setting the position of the coil 21 to a position suitable for treating symptoms in the patient, as described above. To improve the workability of adjusting the position of the coil 21, the magnetic stimulation apparatus 20 is expected to satisfy the following requests.

First, it is easy to adjust the posture of the comparatively heavy coil 21 relative to the arm portion 23.

Second, it is possible to operate the arm portion 23 supporting the comparatively heavy coil 21, free from a sense of the weight of the coil 21.

Third, it is easy to adjust the position to which the arm assembly 500 is slid.

Fourth, it is easy to replace the coil 21. Such requirements will be described below in sequence.

[1. Posture Locking Device for Medical Instrument]

For the first request, a posture locking device 100 for a medical instrument which facilitates adjustment of the posture of the coil 21 relative to the arm portion 23 will be described.

Conventionally, when a medical instrument is supported with its posture adjustable by a support body such as an arm, a ball joint including a ball portion and a rod portion is commonly used. It is also a common practice to lock the posture of the medical instrument relative to the support, using an electromagnetic brake which non-pivotably locks the ball portion of the ball joint into the receiving portion of the support. The electromagnetic brake includes a shoe, a spring, and an electromagnet. The shoe can come into contact with the outer peripheral surface of the ball portion. The spring biases an elastic force to press the shoe against the outer peripheral surface of the ball portion. The electromagnet generates a magnetic force to draw the shoe by attraction in the direction in which the shoe separates from the outer peripheral surface of the ball portion, against the elastic force of the spring. When the electromagnet is energized, the shoe separates from the outer peripheral surface of the ball portion against the elastic force of the spring. The posture of the medical instrument can thus be adjusted. When the electromagnet stops being energized, the shoe is biased by the elastic force of the spring and pressed against the outer peripheral surface of the ball portion. The frictional force between the ball portion and the shoe prevents the ball portion from pivoting due to the self-weight of the medical instrument to lock the posture of the medical instrument relative to the support.

However, since such a structure supports the self-weight of the medical instrument using only friction caused by the force of the spring, it is difficult for this structure to support a comparatively heavy medical instrument. In addition, at the time of unlocking, the medical instrument gets completely free so that the load of the medical instrument rapidly acts on the hand of the operator who is adjusting the posture of the medical instrument. It is, therefore, hard for the operator to finely position the medical instrument.

In the magnetic stimulation apparatus 20 according to the embodiment, the posture locking device 100 for a medical instrument has the following arrangement.

Fig. 3A (A) illustrates a plan view of the posture locking device 100 for a medical instrument while the posture of the medical instrument is kept locked, Fig. 3A(B) illustrates a front view of the posture locking device 100 for a medical instrument while the posture of the medical instrument is kept locked, and Fig. 3B is a sectional view taken along a line 3B - 3B in Fig. 3A(A). Fig. 4A(A) illustrates a plan view of the posture locking device 100 for a medical instrument while the posture of the medical instrument is adjusted finely, Fig. 4A(B) illustrates a front view of the posture locking device 100 for a medical instrument while the posture of the medical instrument is adjusted finely, and Fig. 4B is a sectional view taken along a line 4B - 4B in Fig. 4A (A). Fig. 5 is a side view illustrating the posture locking device 100 for a medical instrument while the posture of the medical instrument is adjusted finely. Fig. 6 is a sectional view illustrating the posture locking device 100 for a medical instrument when biasing with a first resistive force by a first braking portion and biasing with a second resistive force by a second braking portion have been cancelled.

To be brief, the posture locking device 100 for a medical instrument (to be also simply referred to as the "posture locking device 100" hereinafter) according to the embodiment includes a medical instrument and a braking mechanism 104. The medical instrument is connected to a rod portion 102 of a ball joint 43 including a ball portion 101 and the rod portion 102. The braking mechanism 104 non-pivotably locks the ball portion 101 of the ball joint 43 into a receiving portion 103 to, in turn, locks the posture of the medical instrument relative to the support. The braking mechanism 104 includes a first braking portion 105, a second braking portion 106, a first switch 107, and a second switch 108. The first braking portion 105 includes a first shoe 109 which can come into contact with the outer peripheral surface of the ball portion 101. The first braking portion 105 presses the first shoe 109 against the outer peripheral surface of the ball portion 101 to bias a first resistive force f1 as a resistance against pivoting of the ball portion 101 due to the self-weight of the medical instrument. The second braking portion 106 includes a second shoe 110 which can come into contact with an area different from that contacted by the first shoe 109, on the outer peripheral surface of the ball portion 101. The second braking portion 106 presses the second shoe 110 against the outer peripheral surface of the ball portion 101 to bias a second resistive force (f2) as a resistance against pivoting of the ball portion 101 due to the self-weight of the medical instrument. The first switch 107 selectively turns on or off biasing with the first resistive force f1 by the first braking portion 105. The second switch 108 selectively turns on or off biasing with the second resistive force f2 by the second braking portion 106. In finely adjusting the posture of the medical instrument, the posture locking device 100 biases only one of the first resistive force f1 and the second resistive force f2. In locking the posture of the medical instrument, the posture locking device 100 biases both the first resistive force f1 and the second resistive force f2. In the embodiment, the above-mentioned "medical instrument" is implemented in a coil 21 used in a magnetic stimulation treatment which magnetically stimulates a living body (for example, a head). Note, however, that "the self-weight of the medical instrument" described above is defined as the self-weights of both the coil 21 and the coil attachment portion 22 because the coil 21 is connected to the rod portion 102 of the ball joint 43 via the coil attachment portion 22. Details will be described below.

Referring to Fig. 2, the posture locking device 100 is located at the distal end portion of the arm portion 23 corresponding to the support body. The coil 21 is suspended from the arm portion 23 through the ball joint 43.

Referring to Fig. 3A(A), Fig. 3A(B), Fig. 3B, Fig. 4A(A), Fig. 4A(B), and Fig. 4B, the posture lockingdevice 100 includes a hollow cylindrical casing 111 having its open upper end sealed with a cover member 112 including a central hole 112a. A lower disk 113 is mounted on the bottom portion within the casing 111. The lower disk 113 includes a receiving portion 103 which has an arcuate cross-section and is contacted by approximately the lower half of the ball portion 101. The receiving portion 103 includes a through hole 103a in which the rod portion 102 is inserted. An upper disk 114 is accommodated at the position above the lower disk 113 to be capable of coming close to or separating from the lower disk 113. The upper disk 114 includes a first shoe 109 which has an arcuate cross-section and is contacted by approximately the upper half of the ball portion 101. The first shoe 109 includes a central hole 109a in which a shaft 116 of the second braking portion 106 is inserted (to be described later). The ball portion 101 is interposed between the receiving portion 103 of the lower disk 113 and the first shoe 109 of the upper disk 114. The material forming the lower disk 113 and the upper disk 114 is not particularly limited but can be, for example, a highly rigid rubber material. A spring 115 is interposed between the cover member 112 and the upper disk 114. The spring 115 biases an elastic force to press the first shoe 109 against the outer peripheral surface of the ball portion 101.

The first braking portion 105 includes a first shoe 109 and a spring 115. The first shoe 109 can come into contact with the outer peripheral surface of the ball portion 101. The spring 115 is interposed between the cover member 112 and the upper disk 114 and biases an elastic force to press the first shoe 109 against the outer peripheral surface of the ball portion 101. The first braking portion 105 uses the elastic force of the spring 115 to bias the first resistive force f1 as a resistance against pivoting of the ball portion 101 due to the self-weights of both the coil 21 and the coil attachment portion 22.

The second braking portion 106 is implemented in a toggle clamp which uses a toggle mechanism acting as a servomechanism. The second braking portion 106 includes a shaft 116 and a control lever 118. The shaft 116 is inserted into the central hole 109a in the first shoe 109. The control lever 118 is connected to the shaft 116 via a toggle mechanism 117. The shaft 116 includes, at its lower, distal end illustrated in Fig. 3B, a second shoe 110 which can come into contact with the outer peripheral surface of the ball portion 101. The material forming the second shoe 110 is not particularly limitedbutcanbe, for example, a highly rigid rubber material. The second shoe 110 comes into contact with the outer peripheral surface of the ball portion 101 in an area defining the central hole 109a formed in the first shoe 109. In other words, the second shoe 110 can come into contact with an area different from that contacted by the first shoe 109, on the outer peripheral surface of the ball portion 101. When the control lever 118 is tilted downwards as shown in Fig. 3A(B), Fig. 3B, Fig. 4A(B), and Fig. 4B, the toggle mechanism 117 that is one type of link mechanism pushes the shaft 116 down to the ball portion 101 so that the second braking portion 106 biases a force to press the second shoe 110 against the outer peripheral surface of the ball portion 101. The second braking portion 106 uses the force converted by the toggle mechanism 117 to bias the second resistive force f2 as a resistance against pivoting of the ball portion 101 due to the self-weights of both the coil 21 and the coil attachment portion 22.

The first switch 107 includes a releasing cam 119 disposed on the lower surface of the upper disk 114. The releasing cam 119 includes a rotating shaft 120, an eccentric cam 121, and a control lever 122. The eccentric cam 121 is attached to the rotating shaft 120. The control lever 122 is connected to the rotating shaft 120 and arranged at outside of the casing 111. When the control lever 122 pivots from the position shown in Fig. 4A (B), 4B and 5 to that shown in Fig. 3A (B) and Fig. 3B, a projection 121a of the eccentric cam 121 shifts downwards. With this operation, the first braking portion 105 presses the first shoe 109 of the upper disk 114 against the outer peripheral surface of the ball portion 101 using the elastic force of the spring 115 to bias the first resistive force f1. On the other hand, when the control lever 122 pivots from the position shown in Fig. 3A(B) and Fig. 3B to that shown in Fig. 4A(B), Fig. 4B and Fig. 5, the projection 121a of the eccentric cam 121 shifts upwards. With this operation, the first shoe 109 of the upper disk 114 separates from the outer peripheral surface of the ball portion 101 against the elastic force of the spring 115 so that the first braking portion 105 cancels biasing with the first resistive force f1.

The second switch 108 is equipped with the toggle mechanism 117 in the second braking portion 106. When the control lever 118 is tilted downwards to the position shown in Fig. 3A(B), Fig. 3B, Fig. 4A(B) and Fig. 4B, the toggle mechanism 117 pushes the shaft 116 down to the ball portion 101 so that the second braking portion 106 biases the second resistive force f2. On the other hand, when the control lever 118 is tilted upwards to the position shown in Fig. 6, the toggle mechanism 117 pulls up the shaft 116, which moves away from the outer peripheral surface of the ball portion 101 so that the second braking portion 106 cancels biasing with the second resistive force f2. Since the second braking portion 106 uses a toggle clamp, the shaft 116 is easily operated by one action of tilting the control lever 118 downwards or upwards. Unlike a mode in which a knob bolt is turned clockwise or counterclockwise to turn on or of f pressing of the second shoe 110 against the outer peripheral surface of the ball portion 101, it is possible to easily, quickly, selectively turn on or off biasing with the second resistive force f2.

The first switch 107 is connected to an electrical switch (not illustrated). Locked/free of other movable portions (for example, the articulatedportion 44, the bearingportion, and the sliding mechanism) is switched to interlock with the operation of the first switch 107. More specifically, when the first braking portion 105 biases the first resistive force f1, other movable portions are also locked in an inoperable state to interlock with the operation of the first switch 107. This fixes the posture and position of the coil 21. In contrast, in cancelling biasing with the first resistive force f1 by the first braking portion 105, other movable portions are also freed in an operable state to interlock with the operation of the first switch 107. With this operation, the posture and position of the coil 21 can be adjusted by moving the arm assembly 500. Independently of the first switch 107, an operating switch dedicated to switching locked/free of other movable portions (for example, the articulated portion 44, the bearing portion, and the sliding mechanism) can even be used. When a dedicated operating switch is used, the position of the coil 21 can be adjusted by moving other joints of the arm portion 23 while a given posture of the coil 21 is maintained by biasing with the first resistive force f1.

In the embodiment, the posture locking device 100 biases only a biasing, second resistive force f2 (Fig. 4B) in finely adjusting the posture of the coil 21, and biases both the first resistive force f1 and the second resistive force f2 (Fig. 3B) in locking the posture of the coil 21. Even in finely ad justing the posture of the coil 21, the second braking port ion 106 biases the second resistive force f2 so that the coil 21 and the coil attachment portion 22 are not completely free. The operator requires no force to support the self-weights of both the coil 21 and the coil attachment portion 22. This means that a relatively small operating force suffices to adjust the posture of the coil 21, thus improving the workability of finely adjusting the posture of the coil 21. In locking the posture of the coil 21, both a first resistive force f1 and a second resistive force f2 are biased, resulting in an increase in braking force to non-pivotably lock the ball portion 101 into the receiving portion 103. It is, therefore, possible to stably support the comparatively heavy coil 21.

A second resistive force f2 biased in finely adjusting the posture of the coil 21 is preferably smaller than a maximum pivoting force Fmax of pivoting forces to allow the ball portion 101 to pivot due to the self-weights of both the coil 21 and the coil attachment portion 22 at positions that fall within the range in which the coil 21 is movable through the ball joint 43. If the second resistive force f2 is equal to or larger than the maximum pivoting force Fmax, an operating force to adjust the posture of the coil 21 is large. This makes it impossible to finely adjust the posture of the coil 21. If the second resistive force f2 is smaller than the maximum pivoting force Fmax, a relatively small operating force suffices to adjust the posture of the coil 21, although slight movement may occur due to the self-weights of both the coil 21 and the coil attachment portion 22, depending on the posture of the coil 21. This improves the workability of finely adjusting the posture of the coil 21.

Actions will be described below.

When the posture of the coil 21 relative to the armportion 23 is finely adjusted while the posture of the coil 21 is kept locked, the operator uses the first switch 107 to cancel biasing with the first resistive force f1 by the first braking portion 105 while he or she uses the second switch 108 to keep biasing with the second resistive force f2 by the second braking portion 106. More specifically, the control lever 122 of the releasing cam 119 pivots to the position shown in Fig. 4A(B) and Fig. 4B while the control lever 118 of the second braking portion 106 is kept tilted downwards at the position shown in Fig. 4A (B) and Fig. 4B. With this operation, the toggle mechanism 117 keeps the shaft 116 pushed down to the ball portion 101 so that the second braking portion 106 biases the second resistive force f2. Since the projection 121a of the eccentric cam 121 shifts upwards, the first shoe 109 of the upper disk 114 separates from the outer peripheral surface of the ball portion 101 against the elastic force of the spring 115 so that the first braking portion 105 cancels biasing with the first resistive force f1.

The operator sets the position of the coil 21 to a position suitable for treating symptoms in the patient. At this time, the second braking portion 106 biases the second resistive force f2 so that the coil 21 and the coil attachment portion 22 are not completely free. The operator requires no force to support the self-weights of both the coil 21 and the coil attachment portion 22. This means that a relatively small operating force suffices to adjust the posture of the coil 21, thus improving the workability of finely adjusting the posture of the coil 21.

In locking the posture of the coil 21 after the end of fine adjustment of the posture of the coil 21, the operator uses the first switch 107 to bias the first resistive force f1 by the first braking portion 105 while he or she uses the second switch 108 to keep biasing with the second resistive force f2 by the secondbrakingportion 106. More specifically, the control lever 122 of the releasing cam 119 pivots to the position shown in Fig. 3A(B) and Fig. 3B while the control lever 118 of the second braking portion 106 is kept tilted downwards at the position shown in Fig. 3A(B) and Fig. 3B. With this operation, the toggle mechanism 117 keeps the shaft 116 pushed down to the ball portion 101 so that the second braking portion 106 biases the second resistive force f2. Since the projection 121a of the eccentric cam 121 shifts downwards, the first shoe 109 of the upper disk 114 is pressed against the outer peripheral surface of the ball portion 101 by the elastic force of the spring 115 so that the first braking portion 105 biases the first resistive force f1.

In significantly changing the posture of the coil 21 while the posture of the coil 21 is kept locked, the operator uses the second switch 108 to cancel biasing with the second resistive force f2 by the secondbrakingportion 106 and further uses the first switch 107 to cancel biasing with the first resistive force f1 by the first braking portion 105. More specifically, the control lever 118 of the second braking portion 106 is tilted upwards to the position shown in Fig. 6 and the control lever 122 of the releasing cam 119 pivots to the position shown in Fig. 6. With this operation, the toggle mechanism 117 pulls up the shaft 116, which moves away from the outer peripheral surface of the ball portion 101 so that the second braking portion 106 cancels biasing with the second resistive force f2. Since the projection 121a of the eccentric cam 121 shifts upwards, the first shoe 109 of the upper disk 114 separates from the outer peripheral surface of the ball portion 101 against the elastic force of the spring 115 so that the first braking portion 105 cancels biasing with the first resistive force f1.

The operator significantly changes the position of the coil 21. At this time, since biasing with the first resistive force f1 and biasing with the second resistive force f2 have been cancelled, the coil 21 and the coil attachment portion 22 are completely free. Although the operator requires a force to support the self-weights of both the coil 21 and the coil attachment portion 22, no braking force acts to impair the workability of significantly changing the posture of the coil 21.

In another mode, the posture of the coil 21 may be changed by moving the coil 21 along a guide member having a special shape such as a curved shape. However, if the operator uses no such guide member having a special shape, he or she can hardly rely on instinct to perform the operation involved. In the embodiment, the coil 21 is supported by the arm portion 23 through the ball joint 43 used in various machines, the operator can instinctively perform an operation for adjusting the posture of the coil 21.

Fig. 7 is a sectional view illustrating a Modification in which the braking force to lock the posture of the medical instrument is increased.

In the Modification illustrated in Fig. 7, on the outer peripheral surface of the ball portion 101, an area 131 contacted by the first shoe 109 and an area 132 contacted by the second shoe 110 are roughened. The roughening means is not particularly limited. Desired areas can be roughened by, for example, blasting or a surface treatment which irradiates them with a laser beam.

Roughening the outer peripheral surface of the ball portion 101 increases the frictional force between the first shoe 109 and the outer peripheral surface of the ball portion 101 and the frictional force between the second shoe 110 and the outer peripheral surface of the ball portion 101, resulting in a further increase in braking force to lock the posture of the coil 21. It is, therefore, possible to more stably support the comparatively heavy coil 21.

When the rod portion 102 of the ball joint 43 is most greatly tilted with respect to the vertical direction, both the first shoe 109 and the second shoe 110 preferably come into contact with the roughened outer peripheral surface.

Most greatly tilting the rod portion 102 with respect to the vertical direction maximizes the pivoting force to allow the ball portion 101 to pivot due to the self-weights of both the coil 21 and the coil attachment portion 22. Therefore, when the self-weights of both the coil 21 and the coil attachment portion 22 exert an influence the most, setting the braking force to lock the posture of the coil 21 makes it possible to more stably support the comparatively heavy coil 21.

As another Modification in which the braking force to lock the posture of the medical instrument is increased, the ball portion 101 of the ball joint 43 may be formed into a specific shape.

In other words, the ball portion 101 of the ball joint 43 preferably has a non-perfect spherical shape (elliptical cross-section) defined such that the distance between the first shoe 109 and the receiving portion 103 is larger when the rod portion 102 is tilted with respect to the vertical direction than when the rod portion 102 extends vertically.

With such an arrangement, as the self-weights of both the coil 21 and the coil attachment portion 22 are more likely to exert an influence, the distance between the first shoe 109 and the receiving port ion 103 gets larger and the frictional force between the first shoe 109 and the outer peripheral surface of the ball portion 101 gets stronger. Therefore, the comparatively heavy coil 21 can be more stably supported by setting the braking force to lock the posture of the coil 21 stronger.

Similarly, the ball portion 101 of the ball joint 43 preferably has a non-perfect spherical shape (elliptical cross-section) defined such that the distance between the second shoe 110 and the receiving portion 103 is larger when the rod portion 102 is tilted with respect to the vertical direction than when the rod portion 102 extends vertically.

With such an arrangement, as the self-weights of both the coil 21 and the coil attachment portion 22 are more likely to exert an influence, the distance between the second shoe 110 and the receiving portion 103 gets larger and the frictional force between the second shoe 110 and the outer peripheral surface of the ball portion 101 gets stronger. Therefore, the comparatively heavy coil 21 can be more stably supported by setting the braking force to lock the posture of the coil 21 stronger.

As described above, with the posture locking device 100 for a medical instrument according to the embodiment, the braking mechanism 104 includes a first braking portion 105 and a second braking portion 106. The first braking portion 105 and the secondbrakingportion 106 bias the first resistive force f1 and the second resistive force f2 as a resistance against pivoting of the ball portion 101 due to the self-weights of both the coil 21 and the coil attachment portion 22. The braking mechanism 104 biases only the second resistive force f2 in finely adjusting the posture of the coil 21, and biases both the first resistive force f1 and the second resistive force f2 in locking the posture of the coil 21.

With such an arrangement, even in finely adjusting the posture of the coil 21, the second braking portion 106 biases the second resistive force f2 so that the coil 21 and the coil attachment portion 22 are not completely free. The operator requires no force to support the self-weights of both the coil 21 and the coil attachment portion 22. This means that a relatively small operating force suffices to adjust the posture of the coil 21, thus improving the workability of finely adjusting the posture of the coil 21. In locking the posture of the coil 21, both a first resistive force f1 and a second resistive force f2 are biased, resulting in an increase in braking force to non-pivotably lock the ball portion 101 into the receiving portion 103. It is, therefore, possible to stably support the comparatively heavy coil 21.

A second resistive force f2 biased in finely adjusting the posture of the coil 21 is set smaller than a maximum pivoting force (Fmax) of pivoting forces to allow the ball portion 101 to pivot due to the self-weights of both the coil 21 and the coil attachment portion 22 at positions that fall within the range in which the coil 21 is movable through the ball joint 43.

With such an arrangement, a relatively small operating force suffices to adjust the posture of the coil 21, although slight movement may occur due to the self-weights of both the coil 21 and the coil attachment portion 22, depending on the posture of the coil 21. This improves the workability of finely adjusting the posture of the coil 21.

On the outer peripheral surface of the ball portion 101, an area 131 contacted by the first shoe 109 and an area 132 contacted by the second shoe 110 are roughened.

Such an arrangement increases the frictional force between the first shoe 109 and the outer peripheral surface of the ball portion 101 and the frictional force between the second shoe 110 and the outer peripheral surface of the ball portion 101, resulting in a further increase in braking force to lock the posture of the coil 21. It is, therefore, possible to more stably support the comparatively heavy coil 21.

When the rod portion 102 of the ball joint 43 is most greatly tilted with respect to the vertical direction, both the first shoe 109 and the second shoe 110 come into contact with the roughened outer peripheral surface.

With such an arrangement, when the self-weights of both the coil 21 and the coil attachment portion 22 exert an influence the most, the braking force to lock the posture of the coil 21 can be set stronger to more stably support the comparatively heavy coil 21.

The ball portion 101 of the ball joint 43 has a non-perfect spherical shape defined such that the distance between the first shoe 109 and the receiving portion 103 is larger when the rod portion 102 is tilted with respect to the vertical direction than when the rod portion 102 extends vertically.

With such an arrangement, as the self-weights of both the coil 21 and the coil attachment portion 22 are more likely to exert an influence, the braking force to lock the posture of the coil 21 can be set stronger by producing a greater frictional force between the first shoe 109 and the outer peripheral surface of the ball portion 101. It is, therefore, possible to more stably support the comparatively heavy coil 21.

The ball portion 101 of the ball joint 43 has a non-perfect spherical shape defined such that the distance between the second shoe 110 and the receiving portion 103 is larger when the rod portion 102 is tilted with respect to the vertical direction than when the rod portion 102 extends vertically.

With such an arrangement, as the self-weights of both the coil 21 and the coil attachment portion 22 are more likely to exert an influence, the braking force to lock the posture of the coil 21 can be set stronger by producing a greater frictional force between the second shoe 110 and the outer peripheral surface of the ball portion 101. It is, therefore, possible to more stably support the comparatively heavy coil 21.

The coil 21 is suspended from the arm portion 23 through the ball joint 43.

With such an arrangement, the posture of the coil 21 inflicted with the suspension load can be stably supported.

The medical instrument includes a magnetic stimulation coil 21 used in a magnetic stimulation treatment which magnetically stimulates a living body (for example, a head).

With such an arrangement, the position of the coil 21 can be set to a position suitable for treating symptoms in the patient by finely adjusting the posture of the coil 21.

The posture locking device 100 for a medical instrument is not limited to the above-described arrangement and can be modified as appropriate.

The arrangements of the first braking portion 105 and the second braking portion 106 are not limited to the above-described arrangements and can be any appropriate arrangements as long as a first resistive force f1 and a second resistive force f2 to act as braking forces on the ball portion 101 of the ball joint 43 can be biased. For example, the shaft 116 of the second braking portion 106 may be replaced with a spring. Further, a resistive force may be formed using not only the elastic force of a spring but also an electromagnetic force or a fluid pressure.

The first switch 107 and the second switch 108 are not limited to manually-controlled arrangements and may selectively turn on or off biasing with resistive forces using electromagnetic forces or fluid pressures.

[2. Link Mechanism for Arm Portion 23]

For the second request, a link mechanism for an armportion 23 which can operate the arm portion 23 supporting the coil 21, free from a sense of the weight of the coil 21, will be described next.

In the magnetic stimulation apparatus 20 according to the embodiment, the link mechanism for the arm portion 23 has the following arrangement.

Fig. 8A illustrates a side view of the link mechanism for the arm portion 23 according to the embodiment and Fig. 8B illustrates a bottom view of the link mechanism for the arm portion 23 according to the embodiment. Fig. 9 is a view for explaining an effect produced in the direction of action of an elastic force, and Fig. 10 is a graph illustrating an example of an arm torque diagram in the embodiment. Fig. 11 is a side view illustrating a link mechanism for an arm portion 23 according to a comparative example in which only a spring is used, and Fig. 12 is a graph illustrating an example of an arm torque diagram in the comparative example.

Referring to Figs. 11 and 12, the torque Tarm (to be referred to as the "arm holding torque" hereinafter) for holding the arm portion 23 at a predetermined position peaks when the center of gravity of the armportion 23 is horizontally aligned with an arm rotational axis 201. The arm holding torque reduces as the arm portion 23 descends or ascends from this position.

As denoted by symbol "*" illustrated in Fig. 12, a scheme in which the arm portion 23 is supported by a spring (for example, a coil spring or a gas spring) is disadvantageous since the force to support the spring increases as the arm portion 23 descends as the comparative example. This results in a poor balance between the arm holding torque and the spring supporting torque Tspring2. When the joint brake of the arm portion 23 is released, the arm portion 23 leaps due to the force of the spring. This seriously degrades the operability in adjusting the coil to a position to be treated.

Referring to Fig. 8A and Fig. 8B, the link mechanism for the arm portion 23 according to the embodiment makes Tarm nearly equal to Tspring. To be brief, the link mechanism for the arm portion 23 includes the arm portion 23, a non-rotating arm attachment portion 24, a variable-length rod 202, and a fixed-length rod 203. The arm portion 23 holds a medical instrument. The arm portion 23 is attached to the arm attachment portion 24 to be rotatable about the arm rotational axis 201 as a center. The variable-length rod 202 has a variable total length and includes an elastic member 204 which biases an elastic force to turn back against a pressing force. The fixed-length rod 203 has a fixed total length. One end 202a of the variable-length rod 202 is pivotally attached to the arm attachment portion 24, and the other end 202b of the variable-length rod 202 is pivotally connected to one end 203a of the fixed-length rod 203. The fixed-length rod 203 has its other end 203b attached to the arm portion 23 to be movable about the arm rotational axis 201 upon rotation of the arm portion 23. When the center of gravity of the arm portion 23 is horizontally aligned with the arm rotational axis 201, the direction of action Y of an elastic force is set so as to be matched with a tangential direction to the rotational trajectory x4 along which the other end 203b of the fixed-length rod 203 is movable. The medical instrument includes a coil 21 used in a magnetic stimulation treatment which magnetically stimulates a living body (for example, a head). Details will be described below.

The elastic member 204 of the variable-length rod 202 is made of, for example, a compression spring. The elastic member 204 can be made of a gas spring.

An axis which connects one end 202a of the variable-length rod 202 to the arm attachment portion 24 will be referred to as a "spring fixed axis x1" hereinafter. The other end 202b of the variable-length rod 202 is connected to one end 203a of the fixed-length rod 203 and is free to move. An axis defining this movement will be referred to as a "spring free axis x2" hereinafter. The other end 203b of the fixed-length rod 203 is movable upon rotation of the arm portion 23. An axis defining this movement will be referred to as a "spring movable axis x3" hereinafter. The rotational trajectory along which the other end 203b of the fixed-length rod 203 is movable will be referred to as a "spring movable axis rotational trajectory x4" hereinafter.

When the direction of action Y of an elastic force coincides with a tangential direction to the spring movable axis rotational trajectory x4, the total elastic force can be used as a rotating torque, thus maximizing the efficiency. As the armportion 23 rotates from this position, the direction of action Y of an elastic force is shifted from the tangential direction and the tangential force reduces. For example, as shown in Fig. 9, when the direction of action Y of an elastic force is shifted by 60° from the tangential direction, a half of the elastic force serves as a spring supporting force.

The direction of action of an elastic force can be significantly changed during rotation of the arm portion 23, by using the fixed-length rod 2 03 and providing a spring movable axis in the vicinity of the arm rotational axis 201.

When the arm center-of-gravity angle is negative, the required arm holding torque is small. Accordingly, as a greater effect is produced in the direction of action of an elastic force with the increased elastic force, the spring supporting torque gets smaller.

A spring supporting torque Tspring is given by:

$$\text{Tspring} = r \cdot k \delta L \cdot \cos\theta \qquad \qquad ...(1)$$

where r is the radius of the spring movable axis rotational trajectory, k is a spring constant, θL is the spring expansion/contraction length, and θ is the angle of spring action.

A link mechanism for the arm portion 23 which makes Tarm nearly equal to Tspring, as illustrated in Fig. 10, can be attained by placing a spring to satisfy:

$$k\delta L'\cdot\cos\theta'/k\delta L\cdot\cos|\theta 0| < 1 \qquad \ldots (2)$$

where $\delta L'$ is the spring expansion/contraction length and $\theta'$ is the angle of spring action upon movement of the center of gravity of the arm portion 23 from the horizontal position ($\theta 0$).

As described above, with the link mechanism for the arm portion 23 according to the embodiment, one end 202a of the variable-length rod 202, including the elastic member 204, is pivotally attached to the arm attachment portion 24 and the other end 202b of the variable-length rod 202 is pivotally connected to one end 203a of the fixed-length rod 203. The fixed-length rod 203 has its other end 203b attached to the arm portion 23 to be movable about the arm rotational axis 201 upon rotation of the arm portion 23. When the center of gravity of the arm portion 23 is horizontally aligned with the arm rotational axis 201, the direction of action of an elastic force is set so as to be matched with a tangential direction to the rotational trajectory along which the other end 203b of the fixed-length rod 203 is movable. A tolerance can be defined for the tangential direction, using a damper for holding a frictional force or a resistance on the arm rotational axis 201.

With such an arrangement, a holding torque corresponding to the angle of the arm portion 23 can be obtained to enable the arm portion 23 to be operated free from a sense of the weight of the suspension load. The aforementioned handling can easily be practiced for the angle of depression as well. A small, lightweight magnetic stimulation apparatus 20 can be achieved more easily than the scheme that uses a counterweight.

The medical instrument includes a magnetic stimulation coil 21 used in a magnetic stimulation treatment which magnetically stimulates a living body (for example, a head).

With such an arrangement, the arm portion 23 that holds the coil 21 can be operated free from a sense of the weight of the coil 21 to easily set the position of the coil 21 to a position suitable for treating symptoms in the patient.

[3. Sliding Device 300 for Medical Apparatus]

For the third request, a sliding device 300 for a medical apparatus which facilitates adjustment of the position to which the arm assembly 500 is slid will be described next.

Conventionally, in a medical apparatus equipped with an arm assembly including, for example, an arm and a post portion which supports the arm, the arm assembly is slid to adjust the front-to-back position of a medical instrument attached to the arm.

However, depending on the degree of tilt of the medical apparatus, unlocking the position of the arm assembly may cause an unexpected sliding of the arm assembly. If the medical instrument is relatively heavy as well, unlocking the position of the arm assembly may cause an unexpected sliding of the arm assembly toward the center of gravity of the arm. Such unexpected sliding leads to trouble encountered upon positioning of the medical instrument or the like.

In the magnetic stimulation apparatus 20 according to the embodiment, the sliding device 300 for a medical apparatus has the following arrangement.

Fig. 13 is a plan view illustrating the sliding device 300 for a medical apparatus, Fig. 14 is a sectional view illustrating the main part of the internal structure of the post portion 25 in the sliding device 300 for a medical apparatus, and Fig. 15 is a perspective view illustrating a tension applying member.

To be brief, the sliding device 300 for a medical apparatus according to the embodiment(to be also simply referred to as the "sliding device 300" hereinafter) includes a sliding table 301, a belt member 302, one or a plurality of roller members 303, and a tension applying member 304. The arm assembly 500 that holds a medical instrument is attached to the sliding table 301. The arm assembly 500 includes a post portion 25 that supports the arm portion 23, which holds a medical instrument. The belt member 302 is arranged along the direction in which the sliding table 301 moves. The roller members 303 are mounted on the sliding table 301 and have the belt member 302 bridged over them. The tension applying member 304 applies a tension to the belt member 302 is bridged over the roller members 303. The belt member 302 bridged over the roller members 303 biases a load on the sliding table 301 upon sliding. The medical instrument includes a coil 21 used in a magnetic stimulation treatment which magnetically stimulates a living body (for example, a head). Details will be described below.

Referring to Fig. 13, the sliding table 301 moves in the horizontal direction of Fig. 13. The sliding device 300 includes two linear guides 305 which guide sliding of the sliding table 301. The sliding table 301 is bridged over the two linear guides 305. The belt member 302 extends parallel to the linear guides 305.

Referring to Fig. 14, a plurality of (in the example illustrated in Fig. 14, three) roller members 303 are rotatably attached to the sliding table 301. The three roller members 303 include two idle pulleys 303a and 303b and one braking pulley 303c. The braking pulley 303c is positioned between and higher than the two idle pulleys 303a and 303b to form a triangle. Although the idle pulleys 303a and 303b may be omitted, a load can be more easily exerted upon sliding by a larger number of idle pulleys. The belt member 302 is bridged over the braking pulley 303c while being lifted from the two idle pulleys 303a and 303b. The belt member 302 is implemented in a timing belt which can engage with the roller members 303 (idle pulleys 303a and 303b and braking pulley 303c). The belt member 302 can use any type of belt as long as it causes no slipping on the roller members 303 and are not limited to a timing belt. The belt member 302 may employ, for example, a flat belt which can engage with the roller members 303 using a frictional force.

At least one braking pulley 303c of the plurality of roller members 303 includes a locking mechanism 306 which stops its rotation. If one roller member is used, this roller member includes a locking mechanism 306. The locking mechanism 306 is implemented in, for example, an electromagnetic brake equipped with an electromagnetic clutch. When the electromagnetic brake is energized, the lock is disabled so that the braking pulley 303c freely rotates. When the electromagnetic brake stops being energized, the lock is enabled so that the braking pulley 303c stops its rotation. The position of the sliding table 301 can be locked by stopping by the locking mechanism 306 rotation of the roller member 303 (braking pulley 303c) including the locking mechanism 306. The braking pulley 303c and the idle pulleys 303a and 303b rotate upon movement of the sliding table 301.

Referring to Fig. 15, the tension applying member 304 applies a tension to the belt member 302 bridged over the plurality of roller members 303. The tension applying member 304 includes a slider 310 and a driving unit 311. The end portion of the timing belt 302 is fixed to the slider 310. The driving unit 311 moves the slider 310 parallel to the linear guides 305. The other end portion of the timing belt 302 is fixed. The slider 310 includes a lower plate 312 including an elongated hole 312a, and an upper plate 313 screwed into the lowerplate 312. An engagement hole (not illustrated) is formed in the lower plate 312 to engage with a ridged portion 302a of the timing belt 302. The end portion of the timing belt 302 is interposed between the lower plate 312 and the upper plate 313 and fixed to the slider 310 by screwing the upper plate 313 into the lower plate 312. Guide pins 315 fastened to a base 314 of the sliding device 300 are inserted into the elongatedhole 312a in the lower plate 312. The slider 310 is guided by the guide pins 315 to be movable parallel to the linear guides 305. The driving unit 311 is fixed to the base 314 of the sliding device 300 and includes an adjusting bolt 316 fastened to a flange portion 313a of the upper plate 313. Turning the adjusting bolt 316 to move the slider 310 toward the driving unit 311 makes it possible to apply a tension to the timing belt 302. Turning the adjusting bolt 316 counterclockwise to move the slider 310 opposite to the driving unit 311 makes it possible to weaken a tension applied to the timing belt 302.

The tension applying member 304 can apply an adjustable tension to the timing belt 302. The tension is adjusted by turning the adjusting bolt 316 clockwise or counterclockwise as appropriate to adjust the position to which the slider 310 moves. Adjusting the strength of a tension applied to the timing belt 302 by the tension applying member 304 allows adjustment of the magnitude of a load acting upon sliding of the sliding table 301. Since the magnitude of a load acting upon sliding can be adjusted, an operating force to slide the arm assembly 500 can, in turn, be adjusted to a desired magnitude.

Actions will be described below.

In adjusting the position of the coil 21, when the arm assembly 500 is extended to or retracted from the patient, the lockingmechanism306 of the braking pulley 303c is unlocked to slide the arm assembly 500 forwards or backwards and adjust the front-to-back position of the coil 21.

At this time, the timing belt 302 is bent using the three pulleys 303a, 303b, and 303c upon being applied with a tension. The bent timing belt 302 biases a load on the sliding table 301 upon sliding. Because a moderate biasing load is biased on the sliding table 301, the operator can slide the arm assembly 500 with good operation sense.

Even when the magnetic stimulation apparatus 20 is used with only a slight tilt, since a moderate biasing load is biased on the sliding table 301, the arm assembly 500 does not unexpectedly slide even after unlocking of the locking mechanism 306 of the braking pulley 303c. Further, since the coil 21 is a relatively heavy medical instrument, the center-of-gravity position of the arm portion 23 is shifted to the distal end side. Even in such a case, since a moderate biasing load is biased on the sliding table 301, the arm assembly 500 does not unexpectedly slide in the direction of the center of gravity of the arm portion 23 even after unlocking of the locking mechanism 306 of the braking pulley 303c. Without such unexpected sliding, no trouble is encountered upon positioning of the coil 21 or the like.

When the position to which the arm assembly 500 is slid is determined upon the completion of positioning of the coil 21, rotation of the braking pulley 303c is stopped by the locking mechanism 306. This locks the position of the sliding table 301.

As described above, the sliding device 300 for a medical instrument according to the embodiment includes a sliding table 301, a belt member 302, one or a plurality of roller members 303, and a tension applying member 304. The sliding device 300 uses the belt member 302 bridged over the roller members 303, that is, pulleys 303a, 303b, and 303c to bias a load on the sliding table 301 upon sliding.

With such an arrangement, a moderate biasing load is biased on the sliding table 301 so that the operator can slide the arm assembly 500 with good operation sense. Even when the magnetic stimulation apparatus 20 is used with only a slight tilt or the center-of-gravity position of the arm portion 23 is shifted to the distal end side, since a moderate biasing load is biased on the sliding table 301, the arm assembly 500 does not unexpectedly slide. Unexpected sliding can thus be prevented, enabling accurate positioning of the coil 21 or the like.

The tension applying member 304 can apply an adjustable tension. The magnitude of a load acting upon sliding can be adjusted by adjusting the strength of a tension applied to the belt member 302 by the tension applying member 304.

With such an arrangement, since the magnitude of a load acting upon sliding can be adjusted, an operating force to slide the arm assembly 500 can, in turn, be adjusted to a desired magnitude.

In the tension applying member 304, the strength of a tension applied to the belt member 302 is adjusted by turning the adjusting bolt 316 to move the position where the belt member 302 is held.

With such an arrangement, the strength of a tension applied to the belt member 302 can be easily adjusted simply by turning the adjusting bolt 316. It is, therefore, possible to easily adjust the magnitude of a load acting upon sliding and, in turn, to easily adjust an operating force to slide the arm assembly 500 to a desired magnitude.

The belt member 302 is implemented in a belt (a timing belt or a flat belt) which can engage with the roller members 303. The braking pulley 303c includes a locking mechanism 306, which can lock the position of the sliding table 301 by stopping rotation of the braking pulley 303c.

With such an arrangement, the arm assembly 500 can be easily locked at an arbitrary position after the arm assembly 500 is slid.

The medical instrument includes a magnetic stimulation coil 21 used in a magnetic stimulation treatment which magnetically stimulates a living body (for example, a head).

With such an arrangement, the arm assembly 500 can be slid forwards or backwards and locked to set the position of the coil 21 to a position suitable for treating symptoms in the patient.

The sliding device 300 for a medical instrument is not limited to the above-mentioned arrangement and can be modified where necessary.

For example, although the tension applying member 304 is exemplified by a mode in which the position of one end portion of the timingbelt 302 is changed, the present invention is not limited to this mode. A tension may be applied to the timing belt 302 using an arrangement which raises the axis of the braking pulley 303c while the two end portions of the timing belt 302 are fixed in position. In addition, the magnitude of a tension applied can be adjusted by enabling vertical adjustment of the axis position of the braking pulley 303c.

[4. Attaching and Detaching Device 400 for Coil 21]

For the forth request, an attaching and detaching device 400 which facilitates replacement of the coil 21 will be described next.

In a magnetic stimulation treatment, the shape of coil elements used needs to be selected in accordance with the type of neurological disorder. This requires easily replacing a coil mounted in a magnetic stimulation apparatus.

However, the conventional magnetic stimulation apparatus is designed without consideration of a mechanism for easy coil replacement.

Under the circumstances, in the magnetic stimulation apparatus 20 according to the embodiment, the attaching and detaching device 400 for the coil 21 has the following arrangement.

Fig. 16 is a view illustrating the distal end portion of the arm portion 23 in the magnetic stimulation apparatus 20 when viewed from above; and Fig. 17A illustrates a perspective view of the arm portion 23, the coil attachment portion 22, and the magnetic stimulation coil 21 in the magnetic stimulation apparatus 20 before the magnetic stimulation coil 21 is attached to the coil attachment portion 22, Fig. 17B illustrates a perspective view of the arm portion 23, the coil attachment portion 22, and the magnetic stimulation coil 21 in the magnetic stimulation apparatus 20 after the magnetic stimulation coil 21 is attached to the coil attachment portion 22, when viewed in a direction different from that shown in Fig. 17A. Fig. 18A illustrates a sectional view taken along a line 18 - 18 in Fig. 16, and Fig. 17B illustrates an enlarged sectional view of a portion denoted by reference numeral 18B in Fig. 18A; and Fig. 19A illustrates a sectional view taken along a line 19 - 19 in Fig. 16, and Fig. 19B illustrates an enlarged sectional view of a portion denoted by reference numeral 19B in Fig. 19A.

To be brief, the attaching and detaching device 400 for the magnetic stimulation coil 21 (to be also simply referred to as "the attaching and detaching device 400 for the coil 21" hereinafter) according to the embodiment includes the coil 21, a coil attachment portion 22, a first engagement portion 401, a second engagement portion 402, a locking portion 403, and an unlocking portion 404. The coil 21 is used in a magnetic stimulation treatment which magnetically stimulates a living body (for example, a head). The coil attachment portion 22 is supported by the arm portion 23 and holds the magnetic stimulation coil 21. The first engagement portion 401 is formed in the coil 21 and has a rail shape. The second engagement portion 402 is formed in the coil attachment portion 22 and has a rail shape that allows engagement with the first engagement portion 401 by relatively sliding the first engagement portion 401. The locking portion 403 maintains the engaged state between the first engagement portion 401 and the second engagement portion 402. The unlocking portion 404 releases the state in which the first engagement portion 401 and the second engagement portion 402 are engaged with each other in the locking portion 403. Details will be described below.

As described above, the coil attachment portion 22 has its posture adjustable relative to the arm portion 23.

Adjusting the posture of the coil attachment portion 22 relative to the arm portion 23, in turn, adjusts the posture of the coil 21 attached to the coil attachment portion 22 relative to the armportion 23. The use of the coil attachment portion 22 as an intermediary obviates the need to provide the coil 21 itself with a mechanism for posture adjustment, such as a ball joint. This achieves a small, lightweight coil 21.

Referring to Fig. 16, Fig. 17A, Fig. 17B, Fig. 18A, Fig. 18B, Fig. 19A and Fig. 19B, the first engagement portion 401 is formed in the upper face of the casing of the coil 21 and the second engagement portion 402 is formed under the casing of the coil attachment portion 22. The first engagement portion 401 and the second engagement portion 402 have a rail shape that allows engagement by relative sliding (see Fig. 17A, Fig. 18A and Fig. 18B). The first engagement portion 401 and the second engagement portion 402 form a rail shape because of the relatively heavy weight of the coil 21.

The locking portion 403 includes a lug portion 405 and a recessed portion 406 (see Fig. 19A and Fig. 19B). The lug portion 405 is formed in one of the first engagement portion 401 and the second engagement portion 402 (in the example illustrated in Fig. 19B, the second engagement portion 402). The recessed portion 406 is formed in the other of the first engagement portion 401 and the second engagement portion 402 (in the example illustrated in Fig. 19B, the first engagement portion 401) and fits with the lug portion 405.

The unlocking portion 404 includes a pressing portion 407 which relatively separates the first engagement portion 401 and the second engagement portion 402 from each other. The pressing portion 407 relatively separates the first engagement portion 401 and the second engagement portion 402 from each other to release fitting between the lug portion 405 and the recessed portion 406 (see Fig. 17B, Fig. 19A and Fig. 19B).

The pressing portion 407 is unremovably held by the coil attachment portion 22 (see Fig. 19A and Fig. 19B). The pressing portion 407 does not fall off the coil attachment portion 22 even when the coil 21 has not yet been attached to the coil attachment portion 22.

The pressing portion 407 abuts against the second engagement portion 402 of the coil attachment portion 22 without abutting against the first engagement portion 401 of the coil 21 to separate the second engagement portion 402 from the first engagement portion 401 (see Fig. 19A and Fig. 19B). The length of the pressing portion 407 in the pressing direction is set so as not to allow the pressing portion 407 to abut against the first engagement portion 401 of the coil 21.

The first engagement portion 401, the second engagement portion 402, the lockingportion 403, and the unlocking portion 404 are preferably made of a non-metal material. This is because avoiding the use of a metal member such as a screw or a coil spring in fixing the coil 21 prevents degradation in performance of the coil 21.

Actions will be described below.

In replacing the coil 21 already attached to the coil attachment portion 22, the pressing portion 407 of the unlocking portion 404 is pressed (see Fig. 17B, Fig. 19A and Fig. 19B). The pressing portion 407 abuts against the second engagement portion 402 of the coil attachment portion 22 without abutting against the first engagement portion 401 of the coil 21 to separate the second engagement portion 402 from the first engagement portion 401. This releases fitting between the lug portion 405 and the recessed portion 406. Since the pressing portion 407 does not abut against the first engagement portion 401 of the coil 21 even upon pushing, the coil 21 is not pushed out at the time of release of engagement between the first engagement portion 401 and the second engagement portion 402. It is, therefore, possible to prevent the coil 21 from unexpectedly falling off the coil attachment portion 22. The operator removes the coil 21 from the coil attachment portion 22 (see Fig. 17A).

The operator relatively slides the first engagement portion 401 of a new coil 21 along the second engagement portion 402 of the coil attachment portion 22 (see Fig. 17A, Fig. 18AandFig. 18B). After sliding to a predetermined position, the lug portion 405 of the second engagement portion 402 fits into the recessed portion 406 in the first engagement portion 401. With this operation, the locking portion 403 maintains the engaged state between the first engagement portion 401 and the second engagement portion 402.

As described above, the attaching and detaching device 400 for the coil 21 according to the embodiment includes the coil 21, a coil attachment portion 22, a first engagement portion 401, a second engagement portion 402, a locking portion 403, and an unlocking portion 404. The first engagement portion 401 is formed in the coil 21 and has a rail shape. The second engagement portion 402 is formed in the coil attachment portion 22 and has a rail shape that allows engagement with the first engagement portion 401 by relatively sliding the first engagement portion 401.

With such an arrangement, the coil 21 can be easily attached to the coil attachment portion 22 by relatively sliding the first engagement portion 401 of the coil 21 along the second engagement portion 402 of the coil attachment portion 22. Since the locking portion 403 maintains the engaged state between the first engagement portion 401 and the second engagement portion 402, the coil 21 does not fall. In replacing the coil 21, the unlocking portion 404 releases the engaged state between the first engagement portion 401 and the second engagement portion 402 to allow easy removal of the coil 21. The first engagement portion 401, the second engagement portion 402, the locking portion 403, and the unlocking portion 404 can be made of a non-metal material and therefore do not degrade the performance of the coil 21.

The locking portion 403 includes a lug portion 405 on the second engagement portion 402 and a recessed portion 406 in the first engagement portion 401. The unlocking portion 404 uses the pressing portion 407 to relatively separate the first engagement portion 401 and the second engagement portion 402 from each other to release fitting between the lug portion 405 and the recessed portion 406.

With such an arrangement, the locking portion 403 can easily maintain the engaged state between the first engagement portion 401 and the second engagement portion 402, while the unlocking portion 404 can easily release the engaged state between the first engagement portion 401 and the second engagement portion 402.

The pressing portion 407 is unremovably held by the coil attachment portion 22.

With such an arrangement, the pressing portion 407 does not fall off the coil attachment portion 22 even when the coil 21 has not yet been attached to the coil attachment portion 22.

The pressing portion 407 abuts against the second engagement portion 402 of the coil attachment portion 22 without abutting against the first engagement portion 401 of the coil 21 to separate the second engagement portion 402 from the first engagement portion 401.

With such an arrangement, since the pressing portion 407 does not abut against the first engagement portion 401 of the coil 21 even upon pushing, the coil 21 is not pushed out at the time of release of engagement between the first engagement portion 401 and the second engagement portion 402. It is, therefore, possible to prevent the coil 21 from unexpectedly falling off the coil attachment portion 22.

The coil attachment portion 22 has its posture adjustable relative to the arm portion 23. Adjusting the posture of the coil attachment portion 22 relative to the arm portion 23, in turn, adjusts the posture of the coil 21 attached to the coil attachment portion 22 relative to the arm portion 23.

With such an arrangement, the use of the coil attachment portion 22 as an intermediary obviates the need to provide the coil 21 itself with a mechanism for posture adjustment, such as a ball joint. This achieves a small, lightweight coil 21.

The first engagement portion 401, the second engagement portion 402, the locking portion 403, and the unlocking portion 404 are made of a non-metal material.

With such an arrangement, the use of a metal member such as a screw or a coil spring is avoided in fixing the coil 21, thus preventing degradation in performance of the coil 21.

Although a link mechanism for an arm portion according to an embodiment of the present invention has been described above, the present invention is not limited to the above-described embodiment.

For example, the magnetic stimulation coil taken as an example in the above-described embodiment may be replaced with other medical instruments.

Other characteristic technical matters of the present invention will be supplementarily described below.

First, provided is a posture locking device for a medical instrument which facilitates adjustment of the posture of the medical instrument relative to a support. A technique disclosed in Japanese Patent Publication No. 2005-125056 is known as a related-art technique.

(1) A posture locking device for a medical instrument, the device comprising:

a medical instrument connected to a rod portion of a ball joint comprising a ball portion and the rod portion; and
a braking mechanism which non-pivotably locks the ball portion of the ball joint into a receiving portion to, in turn, lock a posture of the medical instrument relative to a support,
the braking mechanism comprising:
a first braking portion which comprises a first shoe configured to come into contact with an outer peripheral surface of the ball portion, and presses the first shoe against the outer peripheral surface of the ball portion to bias the first resistive force (f1) as a resistance against pivoting of the ball portion due to a self-weight of the medical instrument;
a second braking portion which comprises a second shoe configured to come into contact with an area different from an area contacted by the first shoe, on the outer peripheral surface of the ball portion, and presses the second shoe against the outer peripheral surface of the ball portion to bias the second resistive force (f2) as a resistance against pivoting of the ball portion due to the self-weight of the medical instrument;
a first switch which selectively turns on or off biasing with the first resistive force (f1) by the first braking portion; and
a second switch which selectively turns on or off biasing with the second resistive force (f2) by the second braking portion,
wherein in finely adjusting the posture of the medical instrument, the device biases only one of the first resistive force (f1) and the second resistive force (f2), and
in locking the posture of the medical instrument, the device biases both the first resistive force (f1) and the second resistive force (f2).

(2) The posture locking device for a medical instrument according to above (1), in which one of the first resistive force (f1) and the second resistive force (f2) exerted for biasing in finely adjusting the posture of the medical instrument is smaller than a maximum pivoting force (Fmax) of pivoting forces to allow the ball portion to pivot due to the self-weight of the medical instrument at positions that fall within a range in which the medical instrument is movable through the ball joint.

(3) The posture locking device for a medical instrument according to above (1) or (2), in which on the outer peripheral surface of the ball portion, an area contacted by the first shoe and an area contacted by the second shoe have a roughened surface.

(4) The posture locking device for a medical instrument according to above (3), in which when the rod portion of the ball joint is most greatly tilted with respect to a vertical direction, both the first shoe and the second shoe come into contact with the roughened outer peripheral surface.

(5) The posture locking device for a medical instrument according to any one of above (1) to (4), in which the ball portion of the ball joint has a non-perfect spherical shape defined such that a distance between the first shoe and the receiving portion is larger when the rod portion is tilted with respect to the vertical direction than when the rodportion extends vertically.

(6) The posture locking device for a medical instrument according to any one of above (1) to (5), in which the ball portion of the ball joint has a non-perfect spherical shape defined such that a distance between the second shoe and the receiving portion is larger when the rod portion is tilted with respect to the vertical direction than when the rodportion extends vertically.

(7) The posture locking device for a medical instrument according to any one of above (1) to (6), in which the medical instrument is suspended from the support through the ball joint.

(8) The posture locking device for a medical instrument according to any one of above (1) to (7), in which the medical instrument comprises a magnetic stimulation coil used in a magnetic stimulation treatment which magnetically stimulates a living body.

Second, provided is a sliding device for a medical apparatus which facilitates adjustment of the position to which an arm assembly is slid. A technique disclosed in Japanese Patent Publication No. 2008-528108 is known as a related-art technique.

(1) A sliding device for a medical apparatus, the device comprising:

a sliding table equipped with an arm assembly which holds a medical instrument;
a belt member which extends in a direction in which the sliding table moves;
at least one roller member which is mounted on the sliding table and over which the belt member is bridged; and
a tension applying member which applies a tension to the belt member bridged over the roller member,

wherein the belt member bridged over the roller member biases a load on the sliding table upon sliding.
(2) The sliding device for a medical apparatus according to above (1), in which
the tension applying member is configured to apply an adjustable tension, and
adjusting a strength of the tension applied to the belt member by the tension applying member allows adjustment of a magnitude of the load acting upon sliding.
(3) The sliding device for a medical apparatus according to above (1), in which in the tension applying member, the strength of the tension applied to the belt member is adjusted by turning an adjusting bolt to move a position where the belt member is held.
(4) The sliding device for a medical apparatus according to any one of above (1) to (3), in which
the belt member is implemented in a belt engageable with the roller member,
at least one of the at least one roller member comprises a locking mechanism which stops rotation, and
the locking mechanism stops rotation of the roller member provided with the locking mechanism to allow the sliding table to be locked in position.
(5) The sliding device for a medical apparatus according to any one of above (1) to (4), in which the medical instrument comprises a magnetic stimulation coil used in a magnetic stimulation treatment which magnetically stimulates a living body.

Third, provided is an attaching and detaching device which facilitates replacement of a magnetic stimulation coil. A technique disclosed in Japanese Patent Publication No. 2008-528108 is known as a related-art technique.

(1) An attaching and detaching device for a magnetic stimulation coil, the device comprising:

a magnetic stimulation coil used in a magnetic stimulation treatment which magnetically stimulates a living body;
a coil attachment portion which is supported by an arm portion and holds the magnetic stimulation coil;

a first engagement portion which is formed on the magnetic stimulation coil and has a rail shape;

a second engagement portion which is formed on the coil attachment portion and has a rail shape that allows engagement with the first engagement portion by relatively sliding the first engagement portion;

a locking portion which maintains an engaged state between the first engagement portion and the second engagement portion; and

an unlocking portion which releases the engaged state between the first engagement portion and the second engagement portion in the locking portion.

(2) The attaching and detaching device for a magnetic stimulation coil according to above (1), in which the locking portion comprises:

a lugportion formed on one of the first engagement portion and the second engagement portion; and

a recessed portion which is formed in the other of the first engagement portion and the second engagement portion and fits with the lug portion, and

the unlocking portion comprises a pressing portion which relatively separates the first engagement portion and the second engagement portion from each other, and uses the pressing portion to relatively separate the first engagement portion and the second engagement portion from each other to release fitting between the lug portion and the recessed portion.

(3) The attaching and detaching device for a magnetic stimulation coil according to above (2), in which the pressing portion is unremovably held by the coil attachment portion.

(4) The attaching and detaching device for a magnetic stimulation coil according to above (3), in which the pressing portion abuts against the second engagement portion of the coil attachment portion without abutting against the first engagement portion of the magnetic stimulation coil to separate the second engagement portion from the first engagement portion.

(5) The attaching and detaching device for a magnetic stimulation coil according to any one of above (1) to (4), in which the coil attachment portion has a posture adjustable relative to the arm portion, and

a posture of the magnetic stimulation coil attached to the coil attachment portion is adjusted relative to the arm portion by adjusting the posture of the coil attachment portion relative to the arm portion.

(6) The attaching and detaching device for a magnetic stimulation coil according to any one of above (1) to (5), in which the first engagement portion, the second engagement portion, the locking portion, and the unlocking portion are made of a non-metal material.

The entire disclosure of Japanese Patent Application No. 2015-39334 filed on February 27, 2013, Japanese Patent Application No. 2015-39337 filed on February 27, 2013, Japanese Patent Application No. 2015-39340 filed on February 27, 2013, and Japanese Patent Application No. 2015-39344 filed on February 27, 2013 including specifications, claims, drawings and summaries are incorporated herein by reference in its entirety.

**Claims**

1. A link mechanism for an arm portion, the mechanism comprising:

an arm portion which holds a medical instrument;

a non-rotating arm attachment portion to which the arm portion is attached to be rotatable about an arm rotational axis as a center;

a variable-length rod which has a variable total length and comprises an elastic member which biases an elastic force to bring about a recovery against a pressing force; and

a fixed-length rod having a fixed total length;

wherein one end of the variable-length rod is pivotably attached to the arm attachment portion and the other end of the variable-length rod is pivotably connected to one end of the fixed-length rod;

the fixed-length rod has the other end attached to the arm portion to be movable about the arm rotational axis upon rotation of the arm portion; and

wherein when a center of gravity of the arm portion is horizontally aligned with the arm rotational axis, a direction of action of the elastic force is set so as to be matched with a tangential direction to a rotational trajectory along which the other end of the fixed-length rod is movable.

**EP 3 061 494 A1**

2. The link mechanism for an arm portion according to claim 1, in which the medical instrument comprises a magnetic stimulation coil used in a magnetic stimulation treatment which magnetically stimulates a living body.

**18**

FIG.1

FIG.2

## FIG.3A(A)

## FIG.3A(B)

# FIG.3B

## FIG.4A(A)

## FIG.4A(B)

# FIG.4B

FIG.5

108   117

111

122

101

102   43

## FIG.6

FIG.7

# FIG.8A

# FIG.8B

# FIG.9

# FIG.10

◆ ARM HOLDING TORQUE: Tarm
□ SPRING SUPPORTING TORQUE: Tspring

ARM CENTER-OF-GRAVITY ANGLE [°]

# FIG.11

# FIG.12

◆ ARM HOLDING TORQUE: Tarm
■ SPRING SUPPORTING TORQUE: Tspring2

TORQUE

−50                    0                    50

ARM CENTER-OF-GRAVITY ANGLE [°]

# FIG.13

300

# FIG.14

# FIG.15

304

310    311

302

316

302a

312a    315    313

312    313a

314

# FIG.16

## FIG.17A

23

400

402

22

401

21

## FIG.17B

22

404

21

# FIG.18A

# FIG.18B

## FIG.19A

## FIG.19B

Europäisches
Patentamt

European
Patent Office

Office européen
des brevets

**EUROPEAN SEARCH REPORT**

Application Number

EP 16 15 6921

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 91/00472 A1 (WILD HEERBRUGG AG [CH]) 10 January 1991 (1991-01-10) * abstract * * page 5, last paragraph - page 9, paragraph first; figures * | 1,2 | INV. A61N2/02 |
| X | US 2001/027313 A1 (SHIMMURA TORU [JP] ET AL) 4 October 2001 (2001-10-04) * abstract * * paragraphs [0085] - [0095]; figures * | 1,2 | |
| X | EP 2 119 411 A1 (OLYMPUS MEDICAL SYSTEMS CORP [JP]) 18 November 2009 (2009-11-18) * abstract * * paragraphs [0005], [0006], [0008] - [0026], [0052] - [0054], [0057] - [0059]; figures * | 1,2 | |
| A | US 2009/227830 A1 (PILLUTLA RAVI [US] ET AL) 10 September 2009 (2009-09-10) * abstract * * paragraphs [0012] - [0016]; figures * | 1,2 | |
| | | | TECHNICAL FIELDS SEARCHED (IPC) |
| A | WO 2014/098568 A1 (SCHINKEL HOLDING B V H [NL]) 26 June 2014 (2014-06-26) * abstract; figures * | 1 | A61N A61B G02B F16M |

The present search report has been drawn up for all claims

| Place of search | Date of completion of the search | Examiner |
|---|---|---|
| The Hague | 4 July 2016 | Rakotondrajaona, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO FORM 1503 03.82 (P04C01)

## EP 3 061 494 A1

### ANNEX TO THE EUROPEAN SEARCH REPORT
### ON EUROPEAN PATENT APPLICATION NO.

EP 16 15 6921

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2016

| Patent document cited in search report | | Publication date | Patent family member(s) | | Publication date |
|---|---|---|---|---|---|
| WO 9100472 | A1 | 10-01-1991 | DE | 3921857 A1 | 17-01-1991 |
| | | | EP | 0433426 A1 | 26-06-1991 |
| | | | JP | H0747998 B2 | 24-05-1995 |
| | | | JP | H04505798 A | 08-10-1992 |
| | | | US | 5213293 A | 25-05-1993 |
| | | | WO | 9100472 A1 | 10-01-1991 |
| US 2001027313 | A1 | 04-10-2001 | JP | 4222706 B2 | 12-02-2009 |
| | | | JP | 2001258903 A | 25-09-2001 |
| | | | US | 2001027313 A1 | 04-10-2001 |
| EP 2119411 | A1 | 18-11-2009 | AT | 526896 T | 15-10-2011 |
| | | | CN | 101579260 A | 18-11-2009 |
| | | | EP | 2119411 A1 | 18-11-2009 |
| | | | JP | 5350675 B2 | 27-11-2013 |
| | | | JP | 2009273714 A | 26-11-2009 |
| | | | US | 2009283647 A1 | 19-11-2009 |
| US 2009227830 | A1 | 10-09-2009 | AU | 2009223654 A1 | 17-09-2009 |
| | | | CA | 2718275 A1 | 17-09-2009 |
| | | | DK | 2252367 T3 | 21-12-2015 |
| | | | EP | 2252367 A1 | 24-11-2010 |
| | | | ES | 2555003 T3 | 28-12-2015 |
| | | | JP | 5453325 B2 | 26-03-2014 |
| | | | JP | 2011513033 A | 28-04-2011 |
| | | | US | 2009227830 A1 | 10-09-2009 |
| | | | WO | 2009114526 A1 | 17-09-2009 |
| WO 2014098568 | A1 | 26-06-2014 | CA | 2896669 A1 | 26-06-2014 |
| | | | CN | 105190150 A | 23-12-2015 |
| | | | EP | 2935971 A1 | 28-10-2015 |
| | | | EP | 2935973 A1 | 28-10-2015 |
| | | | US | 2015338019 A1 | 26-11-2015 |
| | | | US | 2015344153 A1 | 03-12-2015 |
| | | | WO | 2014098568 A1 | 26-06-2014 |
| | | | WO | 2014098571 A1 | 26-06-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011224387 A
- JP 2005125056 A
- JP 2008528108 A
- JP 2015039334 A

- JP 2015039337 A
- JP 2015039340 A
- JP 2015039344 A